(19) Europäisches Patentamt / European Patent Office / Office européen des brevets

(11) EP 1 399 409 B1

(12) EUROPÄISCHE PATENTSCHRIFT

(45) Veröffentlichungstag und Bekanntmachung des Hinweises auf die Patenterteilung:
**31.10.2012 Patentblatt 2012/44**

(21) Anmeldenummer: **02747325.5**

(22) Anmeldetag: **28.05.2002**

(51) Int Cl.:
***C07C 67/54*** (2006.01) ***C07C 213/10*** (2006.01)
***C07C 219/08*** (2006.01) ***C07C 213/06*** (2006.01)

(86) Internationale Anmeldenummer:
**PCT/EP2002/005821**

(87) Internationale Veröffentlichungsnummer:
**WO 2002/100815 (19.12.2002 Gazette 2002/51)**

(54) **VERFAHREN ZUR HERSTELLUNG VON (METH)ACRYLSÄUREESTERN**

METHOD FOR PRODUCING (METH)ACRYLIC ACID ESTERS

PROCEDE DE PRODUCTION D'ESTERS D'ACIDE (METH)ACRYLIQUE

(84) Benannte Vertragsstaaten:
**AT BE CH CY DE DK ES FI FR GB GR IE IT LI LU MC NL PT SE TR**

(30) Priorität: **08.06.2001 DE 10127941**

(43) Veröffentlichungstag der Anmeldung:
**24.03.2004 Patentblatt 2004/13**

(73) Patentinhaber: **BASF SE**
**67056 Ludwigshafen (DE)**

(72) Erfinder:
- **NESTLER, Gerhard**
  **A-1070 Wien (AT)**
- **RAUH, Ulrich**
  **67227 Frankenthal (DE)**
- **SCHRÖDER, Jürgen**
  **67071 Ludwigshafen (DE)**

(56) Entgegenhaltungen:
**EP-A- 0 906 902**

- **DATABASE WPI Section Ch, Derwent Publications Ltd., London, GB; Class E17, AN 88-300074 XP002088821 & BR 8 701 337 A (CIQUINE CIA PETROQU), 27. September 1988 (1988-09-27)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 399 409 B1

## Beschreibung

Die vorliegende Erfindung betrifft ein Verfahren zur Herstellung von höheren (Meth)acrylsäureestern durch Umesterung eines niederen (Meth)acrylsäureesters mit einem höheren Alkanol und Rückführung des dabei freigesetzten niederen Alkohols in den Herstellungsprozeß des niederen (Meth)acrylsäureesters.

(Meth)acrylsäureester sind wertvolle Ausgangsverbindungen zur Herstellung von Polymeren und Copolymeren, die z. B. als Lacke, Dispersionen oder Klebstoffe Anwendung finden.

Der Begriff (Meth)acrylsäure bzw. (Meth)acrylsäureester steht in dieser Anmeldung für Methacrylsäure und Acrylsäure bzw. Methacrylsäureester und Acrylsäureester.

In Kirk Othmer, Encyclopedia of Chemical Technology, 4th Ed., 1994, Seiten 301 - 302, wird eine Herstellung niederer Acrylester durch Veresterung beschrieben, in der Acrylsäure, Alkanol und Katalysator, wie z.B. Schwefelsäure, mit Rückführströmen in einem Reaktor mit angeschlossener Destillationskolonne, in der der Zielester, überschüssiges Alkanol und das bei der Reaktion gebildete Wasser über Kopf abgetrennt werden, umgesetzt werden. Die Phasen im Destillat werden getrennt und ein Teil der organischen Phase als Rücklauf auf die Destillationskolonne gegeben, der größte Teil wird jedoch in einen Waschturm geführt, in dem Alkanol und Acrylsäure durch Waschen entfernt werden. Diesem Waschprozeß kann gegebenenfalls Base zugesetzt werden, um Spuren von Acrylsäure zu entfernen. Der wasserhaltige Ester wird dann destillativ von Wasser befreit und in einer weiteren Destillationskolonne reindestilliert und wiederum im Destillat die Phasen separiert. Die zurückbleibenden Hochsieder werden in einen Strippapparat geleitet, Wertprodukte abgetrennt und rückgeführt.

Die in dem Prozeß anfallenden wäßrigen Phasen aus dem Destillat der Wasserabtrennung, dem Raffinat der Waschung und der wäßrigen Phase des Destillats der auf dem Reaktor angeschlossenen Destillationskolonne werden vereinigt und in einem Alkoholstripper das darin enthaltene Alkanol und Acrylat abgetrennt und in den Reaktor rückgeführt.

Weiterhin werden Hochsieder aus dem Veresterungsreaktor entnommen, in einem Strippapparat Wertprodukte abdestilliert und in den Veresterungsreaktor rückgeführt.

Im Fall der Veresterung mit Butanol wird das Alkanol mit einer weiteren Azeotropkolonne als Butanol-Butylacrylat-Azeotrop entfernt und in den Reaktor rückgeführt.

In Ullmann's Encyclopedia of Industrial Chemistry, 6th ed, 1999 Electronic Release, Kapitel: Acrylic Acid and Derivatives - Esterification, ist ein Verfahren zur Herstellung höherer Alkylacrylate beschrieben, das in Gegenwart eines organischen Solvens als Schleppmittel und Schwefelsäure als Katalysator ausgeführt wird. Das bei der Reaktion entstehende Wasser wird über eine Azeotropdestillation entfernt.

Das dem Reaktor entnommene Reaktionsgemisch wird mit Alkali neutralisiert und in eine wäßrige und eine organische Phase getrennt. Die organische Phase wird in zwei Destillationskolonnen vom verwendeten organischen Solvens sowie vom Alkanol befreit und der so entstandene Rohester anschließend reindestilliert.

Die im Prozeß anfallenden wäßrigen Phasen werden vereinigt und darin enthaltenes organisches Material abgetrennt und in die Reaktion rückgeführt.

Aus diesen beiden Prozessen ist erkennbar, daß (Meth)acrylsäure von dem korrespondierenden Ester durch eine basische Behandlung abtrennbar ist.

In der DE-C2 232 33 28 wird ein Verfahren zur extraktiven Abtrennung der nicht-umgesetzten Acrylsäure aus den Veresterungsablaugen, die bei der Veresterung von Acrylsäure mit Alkanolen, wie n-Butanol, Isobutanol und 2-Ethylhexanol, anfallen, durch Extraktion mit einem Alkanol-Alkylacrylat-Gemisch beschrieben.

Die wäßrigen Lösungen, die der Extraktion zugeführt werden, sollen die Acrylsäure in freier Form enthalten, d.h. alkalische oder neutrale Ablaugen werden zweckmäßigerweise vor der Extraktion z.B. mit Salz- oder Schwefelsäure angesäuert, so daß die gesamte Acrylsäure in Freiheit gesetzt wird.

Bei der praktischen Durchführung des Verfahrens kann die Menge an Butanol-Butylacrylat-Gemisch zu wäßriger Acrylsäure-Lösung in weiten Bereichen variiert werden. Butanol-Butylacrylat-Gemische der genannten Art extrahieren Acrylsäure weit wirkungsvoller, als es Butylacrylat und Butanol für sich tun.

Zusammengefaßt ist die Veresterung von (Meth)acrylsäure mit einem 1 bis 8 Kohlenstoffatome aufweisenden Alkanol zumeist sauer katalysiert, als Katalysatoren dienen beispielsweise saure oder stark saure Ionenaustauscher, Schwefelsäure oder Sulfonsäuren, wie z.B. para-Toluolsulfonsäure, Methansulfonsäure, Trifluormethansulfonsäure, Benzolsulfonsäure, Xylolsulfonsäure, Naphthalinsulfonsäure oder Dodecylbenzolsulfonsäure. Als Katalysator können dabei bis zu 20 Gew% eingesetzt werden, siehe z.B. DE-A 195 10 891.

Die dabei eingesetzte (Meth)acrylsäure kann beispielsweise zuvor aufgereinigt werden, es kann sich aber auch um eine rohe (Meth)acrylsäure handeln, wie es z.B. in der DE-A 198 51 983 beschrieben ist. Als Reaktor kann eine Kaskade sinnvoll sein, siehe z.B. DE-A 195 36 178.

Das bei der Reaktion entstehende Reaktionswasser wird im allgemeinen destillativ abgetrennt, gegebenenfalls unter Zusatz eines ein Azeotrop mit Wasser bildenden Schleppmittels, wie z.B. Benzol, Toluol oder Cyclohexan, gegebenenfalls in einem Azeotrop mit dem eingesetzten Alkanol. Häufig wird der Zielester jedoch zusammen mit dem bei der Reaktion entstehenden Reaktionswasser und dem Alkanol abdestilliert, siehe z.B. WO 99/23060 oder US 4 280 010.

Wie oben beschrieben, wird das häufig (Meth)acrylsäure enthaltende Destillat zumeist in eine organische und eine wäßrige Phase getrennt.

Die organische Phase kann teilweise, aber auch im wesentlichen vollständig als Rücklauf in die Destillationskolonne rückgeführt werden, zumeist wird sie jedoch zumindest teilweise einer Trennoperation unterworfen, in der das Ausgangsalkanol vom Zielester abgetrennt wird, wobei der Zielester gegebenenfalls weiter aufgereinigt und das Alkanol in die Reaktion rückgeführt werden kann.

Die wäßrige Phase kann zumindest teilweise als Rücklauf in die Kolonne rückgeführt werden, sie kann aber auch direkt ausgeschleust oder einer weiteren Aufarbeitung, in der enthaltene Wertprodukte abgetrennt werden, unterworfen werden.

Das der Reaktionszone entnommene Reaktionsgemisch wird zumeist in eine Trennzone geführt, die meist mindestens eine Rektifikationseinheit aufweist. Gegebenenfalls wird zuvor mit einem geeigneten Lösungsmittel, wie z.B. Wasser, extrahiert, um (Meth)acrylsäure und/oder Katalysator abzutrennen. In der Trennzone können auch weitere Phasentrennungen zwischen einer organischen und einer wäßrigen Phase vorgenommen werden, wie es z.B. in der DE-A 196 04 252 und der DE-A 196 04 253 beschrieben ist.

Im Verlauf des Herstellungs- und/oder Aufarbeitungsprozesses als Hochsieder angefallene Oligomere (Oxiester) können rückgespalten werden, z.B. thermisch und in Gegenwart von Stabilisatoren, wie in der US 3 868 410 beschrieben, oder in Gegenwart von Säure, wie z.B. Dodecylbenzolsulfonsäure, siehe z.B. WO 00/27789, in Gegenwart von (Meth)acrylsäure oder oligomerer (Meth)acrylsäure, siehe z.B. DE-A 195 47 485 und DE-A 195 47 459, und gegebenenfalls zusätzlich in Gegenwart von Wasser, siehe z.B. DE-A 197 01 737. Diese Rückspaltung kann z.B. in einer Reaktionskaskade durchgeführt werden, siehe z.B. CN 1 058 390 und CN 1 063 678.

Die Herstellung von (Meth)acrylsäureestern durch Umesterung in Gegenwart von sauren oder basischen Katalysatoren ist allgemein bekannt.

Als Katalysatoren werden vor allem Titanalkoholate vorgeschlagen, deren Alkylgruppen $C_1$-$C_4$-Alkylreste darstellen, z.B. Tetramethyl-, Tetraethyl-, Tetraisopropyl-, Tetra-n-Propyl, Tetraisobutyl- und Tetra-n-Butyltitanat (siehe z.B. EP-B1 298 867, EP-A2 960 877). Weiterhin werden als Katalysatoren u.a. Titanphenolate (DE-OS 20 08 618), Metallchelatverbindungen von z. B. Hafnium, Titan, Zirkon oder Calcium, Alkali- und Magnesiumalkoholate, organische Zinnverbindungen oder Calcium- und Lithiumverbindungen, beispielsweise Oxide, Hydroxyde, Carbonate oder Halogenide, vorgeschlagen.

Da es sich bei der Umesterung bekanntlich um eine Gleichgewichtsreaktion handelt, muß einer der Ausgangsstoffe im großen Überschuß eingesetzt werden und/oder eines der Reaktionsprodukte aus dem Gleichgewicht entfernt werden, um wirtschaftliche Umsätze zu erzielen. In der Regel wird daher das bei der Umesterung freigesetzte niedere Alkanol $R^1OH$ (siehe Gleichung 1) als die Alkoholkomponente mit dem niedrigsten Siedepunkt destillativ aus dem Gleichgewicht entfernt. Nachteilig ist dabei, daß die freigesetzten Alkanole, üblicherweise Methanol bzw. Ethanol, mit den entsprechenden (Meth)acrylsäureestern (Methyl- bzw. Ethyl(meth)acrylat) ein Azeotrop bilden und somit nicht direkt destillativ trennbar sind.

Zudem enthält das Destillat zumindest Spuren des höheren Alkohols $R^2OH$ und ist infolgedessen auch nicht direkt in den Herstellungsprozeß des niederen (Meth)acrylsäureesters I des niederen Alkanols $R^1OH$ rückführbar.

Aus ökologischen und ökonomischen Gründen ist die Wiederverwendung des abdestillierten Gemischs oder Azeotrops bzw. seiner einzelnen Komponenten (Alkanol und/oder (Meth)acrylsäureester) jedoch vorteilhaft.

Aufgrund der Lage der Siedepunkte und/oder der Ausbildung von Azeotropen besteht dieses Destillat, wie oben erwähnt, in der Regel nicht aus dem reinen niederen Alkanol, sondern ist mit dem niederen (Meth)acrylsäureester sowie gegebenenfalls dem höheren Alkohol verunreinigt.

Im Falle der Herstellung von Dimethylaminoethylacrylat aus n-Butylacrylat und Dimethylaminoethanol fällt beispielsweise ein Destillat an, das vorwiegend aus 5-15 Gew% n-Butylacrylat, 85 - 95 Gew% n-Butanol und 0,01 - 0,5 Gew% Dimethylaminoethanol besteht.

Die Einzelkomponenten haben folgende Siedepunkte (Kp.):

| | |
|---|---|
| n-Butylacrylat | Kp. 146,7 °C |
| n-Butanol Azeotrop n-Butanol/ | Kp. 117,5 °C |
| n-Butylacrylat | Kp. 117 °C |
| Dimethylaminoethanol | Kp. 133,9 °C |

Da aus obigen Gründen eine Verwertung des Destillats erwünscht ist, wirkt sich eine Verunreinigung negativ aus, insbesondere wenn der höhere Alkohol wie hier eine basische Verbindung darstellt, d.h. in diesem Fall eine Aminogruppe enthält. Vor allem die als besonders wirtschaftlich anzustrebende direkte Rückführung in die Synthese des niederen Esters wird dadurch beeinträchtigt (EP-A 906 902, Seite 3, Zeilen 4 bis 16).

Die Verunreinigungen sind wegen des geringen Siedepunktsunterschiedes oder Azeotropbildung z.T. schwer

zu entfernen und können zur Bildung von weiteren Nebenprodukten, z.B. durch Umesterungsreaktionen oder Addition an die Doppelbindung der Ester, führen.

EP-A 906 902 versucht das durch die basischen Verunreinigungen bedingte Problem dadurch zu lösen, daß das alkoholhaltige Destillat entweder direkt oder nach einer zusätzlichen Destillation über ein saures Ionenaustauscherharz geleitet wird. Die basischen, stickstoffhaltigen Verunreinigungen werden durch die sauren Gruppen gebunden und dadurch aus dem Alkanol/Acrylester-Gemisch abgetrennt.

Das in der EP-A2 906 902 beschriebene Verfahren zur Herstellung und Isolierung von Alkylaminoalkyl(meth) acrylaten, besteht im wesentlichen aus folgenden Stufen:

1. Diskontinuierliche Umesterung in Gegenwart des Katalysators Dibutylzinnoxid und des Stabilisators Phenothiazin, wobei die Hauptmenge des Alkylaminoälkanols nach dem Start der Reaktion in der Weise zugegeben wird, daß seine Konzentration im Reaktionsgemisch 25 Mol-% nicht überschreitet.

2. Destillative Abtrennung des bei der Umesterung gebildeten niederen Alkanols als Azeotrop mit dem niederen (Meth)acrylester über eine Kolonne, wobei das Destillat gegebenfalls einer weiteren Destillation unterworfen werden kann.

3. Behandlung des Destillats, das hauptsächlich aus niederem Alkanol und niederem (Meth)acrylat besteht, mit einem sauren Kationenaustauscher. Die basischen Verunreinigungen (Amine), die die Verwendung des Destillats bei der Herstellung des niederen Esters durch Desaktivierung des dabei verwendeten Katalysators verhindern, werden dabei abgetrennt.

4. Destillative Auftrennung des Reaktionsgemisches in der Umesterung in ein Kopfprodukt, hauptsächlich aus Zielester, niederem Alkanol und Ausgangsprodukten bestehend, und ein Sumpfprodukt, das im wesentlichen Katalysator, Stabilisator, Michael-Additionsprodukte und Polymere enthält, und unter Umständen bei der Umesterung erneut eingesetzt werden kann. Verliert der Katalysator seine Aktivität, so wird er entsorgt.
Alternativ kann die Katalysatorabtrennung zweistufig erfolgen, wobei zuerst der niedere (Meth)acrylester über den Kopf einer Kolonne abgetrennt und wieder der Umesterung zugeführt wird. In einer zweiten Destillationskolonne wird der Zielester und verbliebene Leichtsieder als Kopfprodukt abgetrennt und der katalysatorhaltige Sumpf gegebenfalls wieder bei einer Umesterung eingesetzt.

5. Das den Zielester enthaltende Destillat wird in einer weiteren Destillationsstufe in ein Kopfprodukt, enthaltend Aminoalkanol und niederen Ester, das wieder bei der Umesterung eingesetzt werden kann, und ein Sumpfprodukt, das den Zielester enthält, aufgetrennt.

6. Aus dem zielesterhaltigen Sumpf wird schließlich in einem weiteren Destillationsschritt (Reindestillation) der Zielester in einer Reinheit von 99,8 % isoliert.

7. Aus dem Sumpfprodukt der Reindestillation, das noch Zielester enthält, wird in einer Destillation, vorzugsweise einer Dünnschichtdestillaton, ein Teil des Zielesters gewonnen und der Leichtsiederdestillation zugeführt. Das Sumpfprodukt wird entsorgt.

Der Nachteil des Verfahrens besteht u. a. darin,

- daß die Umesterung diskontinuierlich erfolgt,
- daß das Dialkylaminoalkanol über einen langen Zeitraum (4 Stunden) verteilt dem Reaktor zugegeben werden muß,
- daß lange Reaktionszeiten (7 - 8 Stunden) benötigt werden, was die Bildung von Nebenprodukten und von Polymerisat begünstigt,
- daß das Azeotrop technisch aufwendig über ein Ionenaustauscherbett gereinigt werden muß, was durch Erfordernis von Spülungen umweltbelastend ist,
- daß die Ausbeute gering ist (ca. 33 % bezüglich eingesetztem Dimethylaminoethanol, s. Bsp. III-1 in EP-A2 906 902) und
- daß die Rückstände nicht aufgearbeitet werden, um Wertprodukte wiederzugewinnen.

EP-A2 960 877 beschreibt ein kontinuierliches Verfahren zur Herstellung von Dialkylaminoalkyl(meth)acrylaten durch Umesterung von Methyl- oder Ethyl(meth)acrylat mit Dialkylaminoalkanolen in Gegenwart von Tetraethyl-, Tetrabutyl- oder Tetra-(2-ethylhexyl)-titanat. Die Umesterung erfolgt dabei in einem Rührreaktor und die Aufarbeitung des Reaktionsgemisch in folgenden Schritten:

1. Das Reaktionsgemisch wird in einer Destillationseinheit in ein Kopfprodukt, das im wesentlichen den Zielester und die Leichtsieder enthält, und ein Sumpfprodukt, das hauptsächlich aus Schwersiedern, Katalysator und etwas Zielester besteht.

2. Das Sumpfprodukt kann gegebenenfalls in einem Dünnschichtverdampfer gereinigt werden, wobei das Destillat wieder der Umesterung zugeführt wird. Der katalysatorhaltige Sumpfablauf wird ausgeschleust.

3. Das den Zielester enthaltende Kopfprodukt wird in einem weiteren Destillationsschritt in eine Leichtsiederfraktion, die in den Reaktor zurückgeführt wird, und ein Sumpfprodukt, vorwiegend Zielester, aufgetrennt

4. In einem weiteren destillativen Reinigungsschritt wird aus dem Sumpfprodukt der Zielester als Kopfprodukt isoliert (Reinheit 99,8 %). Der anfallende Rückstand wird in die Leichtsiederabtrennung zurückgeführt.

Dieses Verfahren hat u. a. folgende Nachteile:

- Die Umesterung erfolgt in einem aufgrund seiner bewegten Teile reparaturanfälligen Rührreaktor
- Die Alkanolkomponente des Katalysator führt zu Verunreinigungen (s. EP-A2 960 877, Seite 2, Zeilen 49 bis 50)
- Keine Verwertung des am Reaktor abgetrennten Destillats, somit Verlust von Wertprodukten
- Keine Verwertung der anfallenden Hochsieder (z.B. Michael-Addukte)

Um allgemein die Bildung eines Destillats bzw. Azeotrops, das aus dem niederen Alkanol und dem entsprechenden (Meth)acrylsäureester besteht, bei der Umesterung zu vermeiden, wird in verschiedenen Patentschriften (z.B. US 2 406 561, DE-OS 2 145 283, EP-B1 210907) die Verwendung von Hilfsstoffen, die mit den freiwerdenden niederen Alkanolen Heteroazeotrope bilden, wie z.B. Hexan, Cyclohexan, Benzol, vorgeschlagen.

Das bei der Umesterung freigesetzte niedere Alkanol wird dabei mit dem Hilfsstoff als Azeotrop destillativ abgetrennt, wobei sich das Kondensat in zwei Phasen auftrennt. Die Phase, die den Hilfsstoff enthält, wird in die Umesterung zurückgeführt und die mit Hilfsstoff gesättigte Alkanolphase ausgeschleust. Diese Alkanolphase muß jedoch vor einer weiteren Verwertung vom restlichen Hilfsstoff getrennt werden. DE-OS 2 145 283 schlägt z. B. die Trennung des Benzol-Alkanol-Azeotrops durch Molekularsiebe vor. Ein solches Verfahren ist aufwendig und im technischen Maßstab nicht wirtschaftlich.

DE-A 23 17 226 schlägt vor, das aus Alkanol und dem entsprechenden (Meth)acrylsäureester gebildete Azeotrop durch Behandlung mit Wasser über Auswaschen des Alkanols zu trennen. Das Verfahren ist nicht wirtschaftlich, da eine wäßrige Alkanollösung anfällt, die entsorgt bzw. aufgearbeitet werden muß, und die Esterphase vor der Rückführung in die Umesterung getrocknet werden muß.

EP-A2 143 639 empfiehlt die Trennung dieser Azeotrope mit komplexbildenden Salzen, z. B. LiCl, und einem Extraktionsmittel. Das Verfahren ist unwirtschaftlich, da es Abwässer produziert und mehrere Destillationsschritte benötigt.

EP-A1 736 510 schlägt vor, die Trennung des Azeotrops aus (Meth)acrylsäuremethylester und Methanol, sowie gegebenenfalls Wasser, destillativ in Gegenwart eines Lösungsmittels, das ein Azeotrop mit Methanol bildet, z.B. Pentan, Hexan, Heptan oder 2,3-Dimethylbutan, durchzuführen.

Der Einsatz eines zusätzlichen Hilfsstoffes macht jedoch auch dieses Verfahren unwirtschaftlich.

Weiterhin wurden Umesterungsverfahren vorgeschlagen, bei denen keine Azeotrope anfallen.

EP-A2 160 427 schlägt beispielsweise eine Umesterung in Abwesenheit von freiem höherem Alkohol vor. Es wird dabei der niedere (Meth)acrylsäureester mit dem Titanalkoholat des höheren Alkohols umgesetzt, wobei neben dem Zielester das Titanat des niederen Alkanols gebildet wird, welches in einem getrennten Reaktionsschritt mit dem höheren Alkohol wieder zu dem entsprechenden Titanat umgesetzt wird. Aufgrund der benötigten hohen Titanatmengen hat dieses Verfahren keine wirtschaftliche Bedeutung.

Ein weiteres Problem bei der Umesterung stellt die Bildung von Michael-Additionsprodukten dar. Unter Michael-Additionsprodukten werden dabei hier die durch Addition der Alkohole an die Doppelbindung der (Meth)acrylsäureester entstandenen Verbindungen verstanden (EP-A2 906 902, Seiten 8 bis 9).

Es ist allgemein bekannt, daß diese Addition (siehe Gleichung 2) besonders in Anwesenheit von alkalischen Katalysatoren erfolgt (Organikum, 17. Auflage, Seite 506, VEB Deutscher Verlag der Wissenschaften, Berlin 1988).

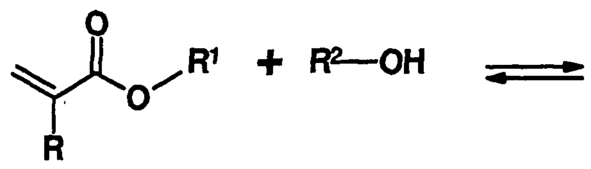

(I)

(Gleichung 2)

Bei der Umesterung entsprechend Gleichung 1 spielen im wesentlichen die Addukte (II) und (III) eine Rolle

(II)

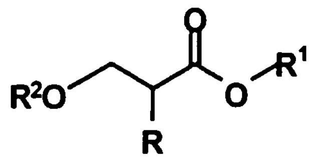

(III)

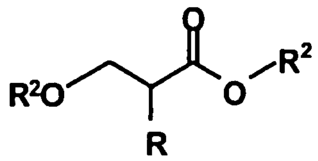

Die Folgen dieser Adduktbildung sind eine verminderte Ausbeute und ein erhöhter Destillationsaufwand um den Zielester in hoher Reinheit zu erlangen.

Die Bildung der Additionsprodukte entsprechend der allgemeinen Gleichung 2 kann, wie allgemein bekannt ist, dadurch verringert werden, daß die Konzentration an freiem Alkanol möglichst niedrig gehalten wird. EP-A2 906 902 schlägt daher vor, die Hauptmenge des Alkanols während der Umesterung kontinuierlich zuzugeben und die Konzentration des freien Alkanols dabei nicht über 25 Mol% ansteigen zu lassen.

Die Aufgabe der vorliegenden Erfindung bestand darin, ein Verfahren zur Herstellung von höheren (Meth) acrylsäureestern durch Umesterung von niederen (Meth)acrylsäureestern zu entwickeln, in dem der freigesetzte niedere Alkohol ohne zusätzliche verfahrenstechnische Schritte in der Herstellung des niederen (Meth)acrylsäureesters wiederverwendet werden kann.

Es wurde nun gefunden, daß man höhere (Meth)acrylsäureester durch Umesterung eines niederen (Meth) acrylsäureesters mit einem höheren Alkohol $R^2OH$, der entweder als $R^2$ einen $C_2$-$C_{12}$-Alkylrest mit mindestens einer $NR^3_2$-Gruppe, in der $R^3$ ein $C_1$-$C_6$-Alkylrest ist und N auch Glied eines fünf- bis siebengliedrigen Ringes sein kann, trägt oder $R^3_2N(-CH_2CH_2-O)_y-H$, $R^3_2N(-CH(CH_3)-CH_2-O)_y-H$ beziehungsweise $R^3_2N(-CH_2CH(CH_3)-O)_y-H$, worin y für eine ganze Zahl zwischen 1 und 4 steht, ist, in Gegenwart eines Stabilisators oder Stabilisatorgemisches und eines Katalysators oder Katalysatorgemischs herstellen kann, wenn man das freigesetzte niedere Alkanol $R^1OH$, wobei $R^1$ mindestens 1 Kohlenstoffatom weniger enthält als $R^2$, abtrennt und ohne weitere Reinigung zumindest teilweise der Herstellung des niederen (Meth)acrylsäureesters zuführt.

Das bei der Umesterung freigesetzte niedere Alkanol $R^1OH$ kann der Herstellung des niederen (Meth)acrylsäureesters I ohne weitere Reinigung zugeführt werden.

Vorzugsweise wird es dabei zur Rückextraktion von (Meth)acrylsäure aus in dem Aufarbeitungsprozeß anfallenden wäßrigen Phasen eingesetzt, besonders bevorzugt bei der wäßrigen Phase, die bei der Abtrennung der überschüssigen (Meth)acrylsäure aus dem Veresterungsgemisch anfällt.

Die Abtrennung des bei der Umesterung freigesetzten niederen Alkanols $R^1OH$ erfolgt dabei bevorzugt destillativ.

Das erfindungsgemäße Verfahren wird im allgemeinen wie folgt durchgeführt:

Gleichung 1

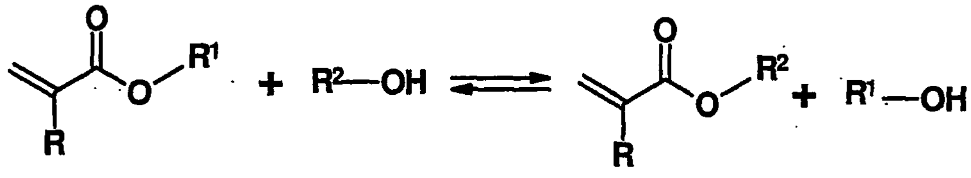

(I) (IV)

$R^1$, $R^2$ und $R^3$ können jeweils aromatisch, aliphatisch oder cycloaliphatisch, geradkettig oder verzweigt, gesättigt oder ungesättigt sein und Heteroatome oder aromatische Substituenten enthalten.

Bevorzugt umfassen die Reste

$R^1$ = $C_1$ - $C_4$ - Alkyl
R = H, $CH_3$
$R^2$ = $C_2$ - $C_{12}$ - Alkyl, substituiert mit mindestens einer $NR^3_2$ - Gruppe, wobei $R^3$ gleich oder verschieden sein kann.
$R^3$ = $C_1$ - $C_6$ - Alkyl, wobei N auch Glied eines fünf- bis siebengliedrigen Ringes sein kann.
$R^1$ soll dabei mindestens ein Kohlenstoffatom weniger enthalten als $R^2$.

Bei $R^1$ handelt es sich beispielsweise um Methyl-, Ethyl-, n-Propyl-, *iso*-Propyl-, n-Butyl-, *iso*-Butyl-, 2-Butyl-, oder *tert*.-Butyl-, bevorzugt um n-Butyl- oder *iso*-Butyl-.

Bei $R^2$ handelt es sich beispielsweise um 2-(Dimethylamino)-ethyl-, 3-(Dimethylamino)-propyl-, 4-(Dimethylamino)-butyl-, 5-(Dimethylamino)-pentyl-, 6-(Dimethylamino)-hexyl-, 8-(Dimethylamino)-octyl-, 10-(Dimethylamino)-decyl-, 22-(Dimethylamino)-dodecyl-, 2-(Diethylamino)-ethyl-, 3-(Diethylamino)-propyl-, 4-(Diethylamino)-butyl-, 5-(Diethylamino)-pentyl-, 6-(Diethylamino)-hexyl-, 8-(Diethylamino)-octyl-, 10-(Diethylamino)-decyl-, 12-(Diethylamino)-dodecyl-, 2-(Di-(*iso*-propyl)-amino)-ethyl-, 3-(Di-(*iso*-propyl)-amino)-propyl-, 4-(Di-(*iso*-propyl)-amino)-butyl-, 5-(Di-(*iso*-propyl)-amino)-pentyl-, 6-(Di-(*iso*-propyl)-amino)-hexyl-, 8-(Di-(*iso*-propyl)-amino)-octyl-, 10-(Di-(*iso*-propyl)-amino)-decyl-, 12-(Di-(*iso*-propyl)-amino)-dodecyl-, 2-(Dibutylamino)-ethyl-, 3-(Dibutylamino)-propyl-, 4-(Dibutylamino)-butyl-, 5-(Dibutylamino)-pentyl-, 6-(Dibutylamino)-hexyl-, 8-(Dibutylamino)-octyl-, 10-(Dibutylamino)-decyl-, 12-(Dibutylamino)-dodecyl-, 2-(Dihexylamino)-ethyl-, 3-(Dihexylamino)-propyl-, 4-(Dihexylaminobutyl-, 5-(Dihexylamino)-pentyl-, 6-(Dihexylamino)-hexyl-, 8-(Dihexylamino)-octyl-, 10-(Dihexylamino)-decyl-, 12-(Dihexylamino)-dodecyl- 2-(Methyl-ethyl-amino)-ethyl-, 2-(Methyl-propylamino)-ethyl-, 2-(Methyl-*iso*-propyl-amino)-ethyl-, 2-(Methyl-butyl-amino)-ethyl-, 2-(Methyl-hexyl-amino)-ethyl-, 2-(Methyl-octyl-amino)-ethyl-, 2-(Ethyl-propyl-amino)-ethyl-, 2-(Ethyl-*iso*-propyl-amino)-ethyl-, 2-(Ethyl-butyl-amino)-ethyl-, 2-(Ethyl-hexyl-amino)-ethyl-, 2-(Ethyl-octyl-amino)-ethyl-, 3-(Methyl-ethylamino)-propyl-, 3-(Methyl-propyl-amino)-propyl-, 3-(Methyl-*iso*-propyl-amino)-propyl-, 3-(Methyl-butyl-amino)-propyl-, 3-(Methylhexyl-amino)-propyl-, 3-(Methyl-octyl-amino)-propyl-, 3-(Ethylpropyl-amino)-propyl-, 3-(Ethyl-*iso*-propyl-amino)-propyl-, 3-(Ethyl-butyl-amino)-propyl-, 3-(Ethyl-hexyl-amino)-propyl-, 3-(Ethyl-octyl-amino)-propyl-, 4-(Methyl-ethyl-amino)-butyl-, 4-(Methyl-propyl-amino)-butyl-, 4-(Methyl-*iso*-propylamino)-butyl-, 4-(Methyl-butyl-amino)-butyl-, 4-(Methyl-hexyl-amino)-butyl-, 4-(Methyl-octyl-amino)-butyl-, 4-(Ethyl-propylamino)-butyl-, 4-(Ethyl-*iso*-propyl-amino)-butyl-, 4-(Ethyl-butylamino)-butyl-, 4-(Ethyl-hexyl-amino)-butyl-, 4-(Ethyl-octylamino)-butyl-, 2-(N-Piperidinyl)-ethyl-, 3-(N-Piperidinyl)-propyl-, 4-(N-Piperidinyl)-butyl-, 5-(N-Piperidinyl)-pentyl-, 6-(N-Piperidinyl)-hexyl-, 8-(N-Piperidinyl)-octyl-, 10-(N-Piperidinyl)-decyl-, 12-(N-Piperidinyl)-dodecyl-, 2-(N-Pyrrolidinyl)-ethyl-, 3-(N-Pyrrolidinyl)-propyl-, 4-(N-Pyrrolidinyl)-butyl-, 5-(N-Pyrrolidinyl)-pentyl-, 6-(N-Pyrrolidinyl)-hexyl-, 8-(N-Pyrrolidinyl)-octyl-, 10-(N-Pyrrolidinyl)-decyl-, 12-(N-Pyrrolidinyl)-dodecyl-, 2-(N-Morpholino)-ethyl-, 3-(N-Morpholino)-propyl-, 4-(N-Morpholino)-butyl-, 5-(N-Morpholino)-pentyl-, 6-(N-Morpholino)-hexyl-, 8-(N-Morpholino)-octyl-, 10-(N-Morpholino)-decyl-, 12-(N-Morpholino)-dodecyl-, 2-(N'-Methyl-N-Piperazinyl)-ethyl-, 3-(N'-Methyl-N-Piperazinyl)-propyl-, 4-(N'-Methyl-N-Piperazinyl)-butyl-, 5-(N'-Methyl-N-Piperazinyl)-pentyl-, 6-(N'-Methyl-N-Piperazinyl)-hexyl-, 8-(N'-Methyl-N-Piperazinyl)-octyl-, 10-(N'-Methyl-N-Piperazinyl)-decyl-, 12-(N'-Methyl-N-Piperazinyl)-dodecyl-, 2-(N'-Ethyl-N-Piperazinyl)-ethyl-, 3-(N'-Ethyl-N-Piperazinyl)-propyl-, 4-(N'-Ethyl-N-Piperazinyl)-butyl-, 5-(N'-Ethyl-N-Piperazinyl)-pentyl-, 6-(N'-Ethyl-N-Piperazinyl)-hexyl-, 8-(N'-Ethyl-N-Piperazinyl)-octyl-, 10-(N'-Ethyl-N-Piperazinyl)-decyl-, 12-(N'-Ethyl-N-Piperazinyl)-dodecyl-, 2-(N'-*iso*-Propyl-N-Piperazinyl)-ethyl-, 3-(N'-*iso*-Propyl-N-Piperazinyl)-propyl-, 4-(N'-*iso*-Propyl-N-Piperazinyl)-butyl-, 5-(*N'*-iso-Propyl-N-Piperazinyl)-pentyl-, 6-(N'-*iso*-Propyl-N-Piperazinyl)-hexyl-, 8-(N'-*iso*-Propyl-N-Piperazinyl)-octyl-, 10-(N'-*iso*-Propyl-N-Piperazinyl)-decyl- oder 12-(N'-*iso*-Propyl-N-Piperazinyl)-dodecyl-.

Weiterhin kann es sich bei $R^2OH$ um ethoxylierte und/oder propoxylierte Alkohole sowie gemischt-ethoxylierte/propoxylierte Aminoalkohole
$R^3_2N(-CH_2CH_2-O)_y-H$ oder

$R^3{}_2N(-CH(CH_3)-CH_2-O)_y-H$ beziehungsweise $R^3{}_2N(-CH_2-CH(CH_3)-O-)_y-H$,
worin y für eine ganze Zahl zwischen 1 und 4 steht,
handeln.

Vorzugsweise werden Dialkylaminoethanole eingesetzt, besonders bevorzugt sind Dimethylaminoethanol, Diethylaminoethanol und Din-butylaminoethanol.

Die Umesterung des niederen (Meth)acrylats (I) mit dem höheren Alkanol $R^2OH$ erfolgt in Gegenwart eines Katalysators oder Katalysatorgemisches auf an sich bekannte Weise, z.B. nach einem der eingangs erwähnten Verfahren.

Typische Bedingungen, unter denen die Umesterung stattfinden kann, sind beispielsweise:

| | |
|---|---|
| niederes (Meth)acrylat : höherer Alkohol $R^2OH$= | 1 - 4 : 1 (molar) |
| Katalysatormenge im Reaktionsgemisch = | 0,5 - 5 Gew. % |
| Stabilisatormenge im Reaktionsgemisch = | 0,05 - 0,5 Gew. % |
| Reaktionstemperatur = | 60 - 160 °C |
| Reaktionsdauer = | 1 - 10 Stdn. |

Die Reaktion kann bei normalem Druck, unter erhöhtem oder vermindertem Druck durchgeführt werden, vorzugsweise bei Atmosphärendruck oder unter leicht vermindertem Druck (300 - 800 mbar absolut).

Die Umesterung kann beispielsweise kontinuierlich, halbkontinuierlich oder diskontinuierlich durchgeführt werden, bevorzugt kontinuierlich.

Als Stabilisatoren werden beispielsweise N-Oxyle, wie z.B. 4-Hydroxy-2,2,6,6-tetramethylpiperidin-N-Oxyl oder 4-Oxo-2,2,6,6-tetramethylpiperidin-N-Oxyl, Phenole und Naphthole, wie z.B. p-Aminophenol, p-Nitrosophenol, 2-*tert*.-Butylphenol, 4-*tert*.-Butylphenol, 2,4-di-*tert*.-Butylphenol, 2-Methyl-4-*tert*.-Butylphenol, 4-Methyl-2,6-*tert*.-Butylphenol (2,6-*tert*.-Butyl-p-Kresol) oder 4-*tert*.-Butyl-2,6-dimethylphenol, Chinone, wie z.B. Hydrochinon oder Hydrochinonmonomethylether, aromatische Amine, wie z.B. N,N-Diphenylamin, Phenylendiamine, wie z.B. N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, z.B. N,N'-Dimethyl-para-phenylendiamin oder N,N'-Diethyl-para-phenylendiamin, Hydroxylamine, wie z.B. N,N-Diethylhydroxylamin, phosphorhaltige Verbindungen, wie z.B. Triphenylphosphin, Triphenylphosphit oder Triethylphosphit oder schwefelhaltige Verbindungen, wie z.B. Diphenylsulfid oder Phenothiazin, oder Gemische davon eingesetzt.

Weiterhin können dies auch Abbauprodukte oder Derivate von Stabilisatoren sein, beispielsweise das Michael-Addukt von (Meth)-acrylsäure beziehungsweise (Meth)acrylsäureester und Hydrochinon.

Die Stabilisierung kann in An- oder Abwesenheit von molekularem Sauerstoff erfolgen.

Bevorzugt erfolgt die Stabilisierung mit Phenothiazin, Hydrochinon, Hydrochinonmonomethylether, 4-Hydroxy-2,2,6,6-tetramethylpiperidin-N-Oxyl, 4-Oxo-2,2,6,6-tetramethylpiperidin-N-Oxyl, 2,6-*tert*.-Butyl-p-Kresol oder Gemischen davon in Mengen von jeweils zwischen 10 und 5000 ppm.

Die Zugabe kann jeweils über die Ausgangsstoffe oder über die Rückführ- oder Rücklaufströme erfolgen.

In besonders bevorzugt Weise wird das gelöste Stabilisatorgemisch auf Kondensatorflächen aufgesprüht.

Als Katalysatoren können die eingangs erwähnten bekannten Umesterungskatalysatoren verwendet werden, bevorzugt Tetraalkyltitanate, (Erd) Alkalimetallalkoholate oder Metallchelate, besonders bevorzugt Tetraalkyltitanate und ganz besonders bevorzugt die Tetraalkyltitanate der an der Reaktion beteiligten Alkohole, also $Ti(OR^1)_4$ oder $Ti(OR^2)_4$.

Wird ein Titanalkoholat $Ti(OR^2)_4$ eingesetzt, so kann dieses vor Einsatz in der Umesterung beispielsweise wie folgt hergestellt werden:

Ein Titanalkoholat $Ti(OR^4)_4$ eines niederen Alkohols, vorzugsweise das Isopropylat, Isobutylat bzw. n-Butylat, wird mit dem höheren Alkohol $R^2OH$ (siehe Gleichung 3) bei erhöhter Temperatur (50 - 130 °C) zur Reaktion gebracht. Dabei wird der höhere Alkohol in molarem Überschuß (1 : 5 bis 1 : 20) eingesetzt. Der niedere Alkohol $R^4OH$ wird während der Reaktion destillativ entfernt.

Gleichung 3 $$\mathbf{Ti(OR^4)_4 + R^2OH \rightleftarrows Ti(OR^2) + R^4OH}$$

$R^2$ s. Gleichung (1)
$R^4$ steht für $C_1$ - $C_8$ - Alkyl, vorzugsweise für Isopropyl, Iso-butyl- oder n-Butyl
$R^2OH$ und $R^4OH$ sollen dabei über ihre Siedepunkte Kp vorzugsweise folgende Bedingung erfüllen:

$$Kp. (R^2OH) \geq Kp. (R^4OH) + 20\ °C$$

Unter diesen Bedingungen ist es technisch einfach, die Verluste an $R^2OH$ gering zu halten und $R^4OH$ möglichst vollständig abzutrennen.

Das bei der Reaktion entstehende Alkanol $R^4OH$ wird, gegebenenfalls bei vermindertem Druck, destillativ oder rektifikativ abgetrennt. Dies kann gegebenenfalls durch Strippen mit einem geeigneten reaktionsträgen Gas unterstützt werden. Der anfallende Rückstand stellt die Katalysatorlösung für die Umesterung dar (Ti - Gehalt: 1-10 Gew%) und enthält in der Regel weniger als 400 ppm $R^4OH$. Es wird somit praktisch kein Fremdalkanol ($R^4OH$) in das Umesterungsgemisch eingeschleust, in der Regel liegt der Gehalt im Gemisch bei weniger als 100 ppm.

Selbstverständlich können in der Katalysatorlösung jedoch auch gemischte Titanalkoholate enthalten sein, in Abhängigkeit der Umsetzung nach Gleichung 3.

Umesterung

Der eingesetzte niedere (Meth)acrylsäureester I hat in der Regel folgende Zusammensetzung:

| | |
|---|---|
| 99,0 - 99,95 Gew% | (Meth)acrylsäureester |
| 0,001 - 0,1 Gew% | Essigsäureester |
| 0,02 - 0,1 Gew% | Propionsäureester |
| 0,001 - 0,05 Gew% | Wasser |

Weiterhin können das niedere Alkanol $R^1OH$, dessen Dialkylether, (Meth)acrylsäure sowie andere, z.B. isomere (Meth)acrylsäureester enthalten sein.

Der höhere Alkohol $R^2OH$ hat üblicherweise eine Reinheit von min. 99,0 Gew% und einen Wassergehalt von 0,01 - 0,2 Gew%.

Der Gehalt an Ethylenglykol im höheren Alkohol $R^2OH$ sollte nicht mehr als 100 ppm betragen, bevorzugt nicht mehr als 50 ppm, besonders bevorzugt nicht mehr als 20 ppm und insbesondere nicht mehr als 10 ppm. Der Gehalt an Vinyloxyethanol im höheren Alkohol $R^2OH$ sollte nicht mehr als 100 ppm betragen, bevorzugt nicht mehr als 50 ppm, besonders bevorzugt nicht mehr als 20 ppm und insbesondere nicht mehr als 10 ppm.

Es können auch Gemische von höheren Alkanolen zur Umesterung verwendet werden.

Der niedere (Meth)acrylsäureester I wird mit dem höheren Alkanol $R^2OH$ in einem molaren Verhältnis von 1 : 1 bis 4 : 1 in Gegenwart mindestens eines der oben beschriebenen Katalysatoren umgesetzt.

Die Umesterung erfolgt in einem Reaktor oder mehreren, bevorzugt in ein bis zwei in Serie geschaltenen Reaktoren mit mindestens einer aufgesetzten Rektifikationskolonne und gegebenenfalls Kondensatoren üblicher Bauart. Dabei kann in einer Kaskade jeder Reaktor eine Destillations- oder Rektifikationskolonne aufweisen oder die Dampfphasen mehrerer Reaktoren werden in mindestens eine gemeinsame Destillations- oder Rektifikationskolonne geleitet. Innerhalb einer Kaskade kann die Temperatur in den einzelnen Behältern gleich oder verschieden sein, bevorzugt wird sie im Verlauf der Kaskade erhöht. Ebenso kann der Druck im Verlauf der Kaskade gleich oder verschieden sein, bevorzugt sinkt er.

Im allgemeinen beträgt die Reaktionstemperatur 60 - 160°C, bevorzugt 80 - 140, besonders bevorzugt 100 - 130 °C, die Gesamtverweilzeit 1-10, bevorzugt 1-8, besonders bevorzugt 2 - 6 und insbesondere 2-4 Stunden.

Die Reaktionszone kann bevorzugt mit einem unter den Reaktionsbedingungen inerten Gas oder Gasgemisch, z.B. Stickstoff, Luft, Stickstoff - Sauerstoff - Gemische, Argon, Helium, Kohlenstoffdi- oder -monooxid) kontinuierlich gespült werden. Besonders bevorzugt wird das Spülgas entlang der vorhandenen Wärmetauscherflächen geleitet, insbesondere in einem vorhandenen Umpump- oder Naturumlaufkreislauf, wie in der deutschen Patentanmeldung mit dem Aktenzeichen DE 101 27 938.8 beschrieben ist.

Eine weitere bevorzugte Ausführungsform der Umesterung kann darin bestehen, daß das Reaktionsgemisch kontinuierlich in einen Nachreäktor geführt wird der gasseitig mit dem Umesterungsreaktor oder der aufgesetzten Kolonne verbunden ist, wie in der gleichen Schrift beschrieben ist.

Die Temperatur im Nachreaktor liegt 1 - 10 °C höher als im Reaktor.

Die Rektifikationskolonnen sind von bekannter Bauart und weisen trennwirksame Einbauten (z. B. Glocken-, Thormann-, Ventil-, Sieb- oder Dual-Flow-Böden) auf oder enthalten Schüttungen oder gerichtete Packungen. Die dem/den Reaktor(en) aufgesetzte(n) Kolonne(n) hat/haben in der Regel 10-30 theoretische böden. Das Rücklaufverhältnis liegt in der Regel bei 5-15 : 1, vorzugsweise bei 7.- 12 : 1. Die Kondensatoren sind ebenfalls von bekannter Bauart, z. B. Röhren- oder Plattenwärmetauscher.

Die gleichmäßige Durchmischung der Reaktionslösung erfolgt auf bekannte Weise, z.B. durch Rühren, Umpumpen oder Naturumlauf. Die Wärmezufuhr kann beispielsweise über eine Doppelwandheizung und/oder außen- oder innenliegende Wärmetauscher, z. B. Rohrbündel- oder Plattenwärmetauscher, erfolgen.

Das bei der Umesterung freigesetzte niedere Alkanol wird dampfförmig über eine aufgesetzte Destillationskolonne aus der Reaktionszone entfernt und gegebenenfalls mit Hilfe eines herkömmlichen Kondensators, z.B. eines Rohrbündel- oder Plattenkondensators, kondensiert. Als Kühlmedium kann beispielsweise Wasser, Luft oder Sole verwendet werden.

Das Verfahren wird dabei bevorzugt so ausgeführt, daß das bei der Umesterung freigesetzte niedere Alkanol $R^1OH$ im wesentlichen zusammen mit niederem (Meth)acrylsäureester I sowie gegebenenfalls höherem Alkohol $R^2OH$ abgetrennt wird.

Die Destillationsbedingungen werden dazu so eingestellt, daß der Alkanolgehalt im Kondensat im Falle von Methanol 20 - 40 Gew.%, im Falle von Ethanol 30 - 65 Gew.%, vorzugsweise 40 - 60 Gew.% und im Fall von n-Butanol 70 bis 95 Gew.%, bevorzugt 80 bis 90 Gew.% beträgt. In der Regel sind mehr als 1, bevorzugt nicht mehr als 0,5 und besonders bevorzugt nicht mehr als 0,3 Gew.% des höheren Alkohols $R^2OH$ enthalten.

In einer besonders bevorzugt Ausführungsform werden die Destillationsbedingungen, z.B. Trennstufen und Rücklaufverhältnis, dabei so gewählt, daß am Kopf der Kolonne ein nichtazeotropes Gemisch angenommen wird, bei dem gegenüber der azeotropen Zusammensetzung aus niederem Alkanol und niederem (Meth)acrylsäureester der Gehalt an niederem (Meth)acrylsäureester erhöht ist.

Das Kondensat kann mit einer Lösung von mindestens eines Stabilisators (s.o.) in der oben angegebenen Menge stabilisiert werden, bevorzugt durch Aufsprühen auf die Kondensatorflächen.

Ein Teil des Destillats, beispielsweise 50 - 95 Gew%, kann wieder in die Destillationskolonne als Rücklauf zurückgeführt werden, der restliche Teil, beispielsweise 5 bis 50 Gew%, wird erfindungsgemäß der Herstellung des niederen Alkyl(meth)acrylats zugeführt. Bevorzugt werden zwischen 60 und 95 Gew% als Rücklauf auf die Destillationskolonne gegeben und zwischen 5 und 40 Gew% der Herstellung des niederen Alkyl(meth)acrylats zugeführt, besonders bevorzugt werden 80 - 95 Gew% als Rücklauf und 5-20 Gew% in die Herstellung geführt.

Selbstverständlich kann das dampfförmige Destillat auch ohne oder nach lediglich partieller Kondensation in die Herstellung des niederen (Meth)acrylsäurealkylesters eingeleitet werden.

Dabei kann das bei der Umesterung abgetrennte Destillat einem Verfahren zur Herstellung des niederen (Meth) acrylsäurealkylesters kontinuierlich zugeführt werden, besonders bevorzugt wird es dem Aufarbeitungsprozeß zugeführt, es kann aber auch getrennt aufgefangen und diskontinuierlich oder halbkontinuierlich zugeführt werden.

Beispielsweise kann das bei der Umesterung abgetrennte Destillat direkt in die Veresterungsreaktion zur Herstellung des niederen (Meth)acrylsäurealkylesters eingespeist werden. Da diese im allgemeinen sauer katalysiert ist, wird der geringe Anteil des höheren, basischen Alkohols protoniert und nimmt dann nicht mehr an der Veresterungsreaktion teil. Diese Zuführung ist bevorzugt, wenn lediglich ein sehr geringer Anteil an höherem Alkanol im Destillat enthalten ist, beispielsweise unter 1 Gew%, bevorzugt unter 0,5 Gew% und besonders bevorzugt unter 0,3 Gew%.

Als Aufarbeitungsprozeß werden dabei die Maßnahmen verstanden, die zur Reinigung des niederen (Meth) acrylsäureesters nach Verlassen der Reaktionszone samt gegebenenfalls aufgesetzter Destillationskolonne erforderlich sind. Dies umfaßt beispielsweise destillative, rektifikative sowie extraktive Schritte.

Das aus der Umesterung stammende Destillat kann beispielsweise

- einem Rektifikationsprozeß, in dem das niedere Alkanol $R^1OH$ vom niederen (Meth)acrylsäureester abgetrennt wird, beispielsweise der Leichtsiederabtrennung des von den Säuren befreiten Veresterungsgemisches in der Herstellung des niederen (Meth)acrylsäureesters, oder

- einem waschprozeß, beispielsweise zum Entfernen von niederem Alkanol, (Meth)acrylsäure und/oder saurem Veresterungskatalysator aus einer organischen Phase mittels einer wäßrigen Phase, z.B. Wasser oder Alkalilauge, zugeführt werden, oder

- zur Rückextraktion von (Meth)acrylsäure aus in dem Aufarbeitungsprozeß anfallenden wäßrigen Phasen eingesetzt werden, beispielsweise aus einer wäßrigen Phase, z.B. einer Veresterungsablauge, die bei der Abtrennung von (Meth)acrylsäure aus dem Veresterungsgemisch anfällt oder einer wäßrigen Phase, die nach Phasentrennung des Destillats bei einer Destillation innerhalb des Aufarbeitungsprozesses anfällt.

Der Aufarbeitungsprozeß der Veresterung kann selbstverständlich auch in Gegenwart eines Hilfsstoffes zur azeotropen Destillation durchgeführt werden, beispielsweise Benzol, Toluol oder Cyclohexan.

Die für die Rückextraktion eingesetzten wäßrigen Phasen können vorteilhafterweise vor der Rückextraktion, z.B. mit Schwefelsäure, angesäuert werden.

Die nach dem Waschen oder der Rückextraktion verbleibenden wäßrigen Phasen können weiter aufgearbeitet, z.B. wiederholt dem erfindungsgemäßen Verfahren unterworfen oder zur Abtrennung von enthaltenem niederen Alkohol oder anderen Wertprodukten destilliert oder gestrippt werden, oder in üblicher Weise entsorgt werden.

Bevorzugt wird das aus der Umesterung stammende Destillat zur Rückextraktion von (Meth)acrylsäure aus im Aufarbeitungsprozeß der Veresterung anfallenden, gegebenenfalls vereinten, wäßrigen Phasen eingesetzt, besonders

bevorzugt bei der Rückextraktion von (Meth)acrylsäure aus Waschwasser, das beim Behandeln des Reaktoraustrages der Veresterung mit einer wäßrigen Phase anfällt.

Dazu wird ein Gewichtsteil der zu extrahierenden wäßrigen Phase mit z.B. 0,1 bis 5 Gewichtsteilen, bevorzugt 0,1 bis 3, besonders bevorzugt,0,15 bis 2 und insbesondere 0,2 bis 1,5 Gewichtsteilen des aus der Umesterung stammenden Destillats behandelt.

Das Destillat kann als solches zur Rückextraktion verwendet werden, es kann jedoch auch niederes Alkanol $R^1OH$ und/oder niederer (Meth)acrylsäurealkylester I zugesetzt werden, so daß das Mischverhältnis im Extraktionsmittel im wesentlichen 1 bis 20 Gewichtsteile niederes Alkanol zu 1 bis 5 Gewichtsteilen niederem (Meth)acrylsäurealkylester beträgt. Ein solcher Zusatz zu dem aus der Umesterung stammenden Destillat ist in der Regel dann vorteilhaft, wenn der Gehalt an niederem (Meth)acrylsäurealkylester unter 30 Gew% liegt.

Die Extraktion wird im allgemeinen bei Temperaturen zwischen dem höchstliegenden Festpunkt und dem niedrigsten Siedepunkt der im System befindlichen Komponenten durchgeführt, beispielsweise zwischen 0°C und 80°C, bevorzugt 0°C bis 60°C und besonders bevorzugt zwischen 10°C und 50°C.

Verfahrenstechnisch können für eine Extraktion nach dem erfindungsgemäßen Verfahren alle bekannten Extraktionsverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt mehrstufige Extraktionen, sowie solche in Gleich- oder Gegenstromfahrweise, bevorzugt Gegenstromfahrweise sein. Vorzugsweise werden Siebboden- oder gepackte beziehungsweise Füllkörperkolonnen oder Mixer-Settler-Apparate, sowie Kolonnen mit rotierenden Einbauten eingesetzt.

In einer weiteren bevorzugten Ausführungsform wird ein beliebiges, bei der Herstellung des niederen Alkyl (meth)acrylats anfallendes (meth)acrylsäurehaltiges organisches Veresterungsgemisch mit zumindest einem Teil der organischen Phase des bei der Umesterung anfallenden Destillats, das nicht als Rücklauf für die Destillationskolonne verwendet wurde, versetzt. Dies kann beispielsweise vor oder während einer Waschung des (meth)acrylsäurehaltigen Stroms mit Wasser und/oder wäßriger Alkalilösung erfolgen.

In einer weiteren bevorzugten Ausführungsform können die Abwässer dieser Säureabtrennung, die gegebenenfalls auch mehrfach durchgeführt werden kann, vereinigt und angesäuert (pH < 3), z.B. mit 20 - 60%-iger Schwefelsäure, werden. Anschließend kann erfindungsgemäß unter Anwendung der Lehre der DE 23 23 328 mit einem Gemisch aus niederem Alkohol und niederem Alkyl(meth)acrylat, z.B. Butanol und Butylacrylat, die (Meth)acrylsäure extrahiert werden, wobei diese Rückextraktion erfindungsgemäß zumindest zum Teil mit dem aus der Umesterung stammenden Destillat erfolgt.

Die (meth)acrylsäurehaltige organische Phase (Extrakt) wird direkt einem, bevorzugt dem ersten Reaktor der Reaktionszone des Veresterungsprozesses, vorzugsweise über eine gegebenenfalls aufgesetzte Destillationskolonne, zugeführt, wobei der Zulauf bevorzugt in der unteren Hälfte der Kolonne erfolgt.

Die anfallenden Abwässer des Herstellprozesses der Veresterung können vereinigt und das darin gelöste niedere Alkohol durch Strippen mit Wasserdampf oder Destillation zurückgewonnen und/oder entsorgt werden.

Die weitere Aufarbeitung des Reaktionsgemisches der Umesterung hat auf das erfindungsgemäße Verfahren im wesentlichen keine Auswirkung.

Sie kann beispielsweise durchgeführt werden wie in den deutschen Patentanmeldungen mit den Aktenzeichen DE 101 27 939.6 und DE 101 27 938.8 beschrieben ist.

Die Herstellung des niederen (Meth)acrylsäureesters ist erfindungsgemäß nicht beschränkt und kann beispielsweise gemäß einem der eingangs erwähnten Verfahren erfolgen, z.B. gemäß der DE-A1 198 51 983. Das dort beschriebene Verfahren besteht im wesentlichen darin, daß der Reaktoraustrag einer Veresterungsreaktion, im wesentlichen aus Zielester, (Meth)acrylsäure, Leichtsieder, Katalysator und Oxyester bestehend, mit Wasser und/oder wäßriger Alkalilösung gewaschen wird, wobei der Katalysator und die nicht umgesetzte (Meth)acrylsäure weitgehend vollständig abgetrennt werden.

Die erfindungsgemäß hergestellten Dialkylaminoalkyl(meth)acrylate, besonders Dialkylaminoethyl(meth)acrylate und speziell Dimethylaminoethyl(meth)acrylate sind wertvolle Monomere für die Herstellung von Copolymerisaten. Als Monomere werden sie in der vorliegenden Form oder nach Quaternisierung in die Polymerisation eingesetzt.

Übliche Quaternisierungsmittel sind beispielsweise Benzylhalogenide wie z.B. Benzylchlorid, Alkylhalogenide wie z.B. Methylchlorid, Ethylchlorid, Methylbromid, Ethylendichlorid oder Allylchlorid, Alkylenoxide wie z.B. Ethylenoxid, Propylenoxid, Styroloxid, *iso*-Butylenoxid oder Vinyloxiran, bevorzugt Ethylenoxid oder Propylenoxid und besonders bevorzugt Ethylenoxid, Alkylphosphite oder -phosphonate wie z.B. Trimethylphosphit oder Triethylphosphit, Dialkylsulfate wie z.B. Dimethylsulfat oder Diethylsulfat, Dialkylcarbonate wie z.B. Dimethylcarbonat, Diethylcarbonat oder Di-n-butylcarbonat, Chlorhydrin oder Epichlorhydrin.

Besonders solche Copolymere, die quaternisierte Monomere einpolymerisiert enthalten, finden Verwendung in der Wasseraufbereitung, beispielsweise als Ionentauscherharze oder als Bestandteil von Membranen.

Das erfindungsgemäße Verfahren wird durch die nachfolgenden Beispiele näher erläutert, ohne es darauf einzuschränken.

In dieser Schrift verwendete ppm- und Prozentangaben beziehen sich, falls nicht anders angegeben, auf Gewichtsprozente und -ppm.

Beispiel 1

Umesterungsbeispiel:

In einem 10 1 Rührreaktor mit aufgesetzter Füllkörperkolonne (Höhe 150 cm, Durchmesser 2,8 cm, Füllkörper 0,5 cm Raschig-Ringe) und Kondensator wurde ein Gemisch aus 3600 g n-Butylacrylat, 1500 g Dimethylaminoethanol, 100 g Titantetra-n-butylat, 3 g Hydrochinonmonomethylether und 1 g Phenothiazin zum Sieden erhitzt. Das freigesetzte n-Butanol wurde gasförmig über die Kolonne abgetrennt und kondensiert (Kopftemperatur 92°C, 370 mbar). Es wurden 1535 g Destillat innerhalb von 4 Stunden ausgeschleust. Auf den Kopf der Kolonne wurden stündlich 100 g einer Lösung von 1000 ppm Phenothiazin in n-Butylacrylat aufgebracht. Das Destillat hatte im wesentlichen folgende Zusammensetzung:

| | |
|---|---|
| 79,5 Gew% | n-Butanol |
| 20,1 Gew% | n-Butylacrylat |
| 0,2 Gew% | Dimethylaminoethanol |

Das Reaktionsgemisch (4060 g) enthielt entsprechend der gaschromatographischen Analyse im wesentlichen

| | |
|---|---|
| 56,3 Gew% | Dimethylaminoethylacrylat |
| 40,1 Gew% | n-Butylacrylat |
| 0,6 Gew% | Dimethylaminoethanol |
| 1,8 Gew% | n-Butanol |
| 0,8 Gew% | Oxyester |

Der Umsatz bezüglich Dimethylaminoethanol betrug 98 %, die Ausbeute 95 %.

Beispiel 2

Extraktionsbeispiel:

Die bei der Herstellung von n-Butylacrylat nach DE-A 198 51 983, Beispiel 2, im Zuge der Säureabtrennung aus dem Reaktionsaustrag anfallenden Ablaugen wurden vereinigt und mit Schwefelsäure angesäuert (pH 1) und bei 25°C mit dem aus dem Umesterungsreaktor abgetrennten Destillat aus Beispiel 1 bzw. mit einem mit n-Butylacrylat angereicherten Destillat beziehungsweise mit Gemischen aus n-Butanol und n-Butylacrylat (je 25 g/100 g Ablauge) die Acrylsäure (Gehalt 3,24 Gew%) einstufig im Scheidetrichter extrahiert.

Ermittelt wurde der Anteil der extrahierten Acrylsäure in der Ablauge.

Extraktionsgrad = (Gehalt an Acrylsäure vor Extraktion - Gehalt an Acrylsäure nach Extraktion) / Gehalt an Acrylsäure vor Extraktion

Das bei der Umesterung von Butylacrylat mit Dimethylaminoethanol anfallende Destillat aus Beispiel 1 hatte im wesentlichen folgende Zusammensetzung:

| | |
|---|---|
| 79,5 Gew% | n-Butanol |
| 20,1 Gew% | n-Butylacrylat |
| 0,2 Gew% | Dimethylaminoethanol |

Zum Vergleich wurden synthetische Gemische aus n-Butanol und n-Butylacrylat mit folgenden Gewichtsverhältnissen eingesetzt:

| Extraktionsmittel: | Extraktionsgrad: |
|---|---|
| Destillat (s. o.) | 35 % |
| Destillat + n-Butylacrylat 1 : 0,6 | 45 % |
| n-Butanol : n-Butylacrylat 1 : 3 | 44 % |
| n-Butanol : n-Butylacrylat 1 : 1 | 45 % |
| n-Butanol : n-Butylacrylat 3 : 1 | 38 % |
| n-Butanol : n-Butylacrylat 4 : 1 | 36 % |

Nach der Extraktion konnte im Extraktionsmittel kein Dimethylaminoethanol nachgewiesen werden.

Beispiel 3

Veresterungsbeispiel:

Eine Rührkesselkaskade bestehend aus 3 Rührreaktoren mit je 11 Reaktionsvolumen, die mit Kolonne, Kondensator und Phäsentrenngefäß ausgerüstet sind, wurde im kontinuierlichen Betrieb pro Stunde mit 533 g Rohacrylsäure, 15 g Schwefelsäure, 510 g n-Butanol und 200 g des bei der Rückextraktion der Acrylsäure anfallenden Gemisches (siehe unten) beschickt. Über die Kolonne des ersten Reaktors wurden außerdem 107 g einer Leichtsiederfraktion (siehe unten) pro Stunde rückgeführt. Die Rohacrylsäure enthielt im wesentlichen 99,3 Gew% Acrylsäure, 0,2 Gew% Essigsäure, 0,03 Gew% Propionsäure, 0,11 Gew% Maleinsäureanhydrid, 0,2 Gew% Diacrylsäure und 0,1 Gew% Phenothiazin. Die Reaktionstemperatur in den Reaktoren betrug 107°C, 118°C und 125°C, der Druck 700 mbar. Am Kopf der Kolonne fiel ein Gemisch aus Wasser, n-Butanol, n-Butylacrylat, n-Butylacetat und Dibutylether an, das in eine wäßrige Phase und eine organische Phase zerfiel. Die wäßrige Phase wurde ausgeschleust, die organische Phase wurde bis auf den ausgeschleusten Anteil (siehe unten) als Rücklauf wieder der Kolonne zugeführt. Von der organischen Phase, hauptsächlich aus 24 Gew% n-Butylacrylat, 54 Gew% n-Butanol, 8 Gew% n-Butylacetat und 2 Gew% Dibutylether bestehend, wurden pro Stunde 25 g ausgeschleust. Die Stabilisierung der Kolonne erfolgte durch Zugabe von 30 g einer 1%-igen Phenothiazinlösung in n-Butylacrylat auf den obersten Boden. Das Kondensat wurde durch Aufbringung von 50 ml einer 1%-igen wäßrigen Lösung von 4-Hydroxy-2,2,6,6-Tetramethylpiperidin-N-oxyl auf den Kondensator stabilisiert. Der Reaktoraustrag (1280 g/h) enthielt noch 0,6 Gew% Acrylsäure, der Acrylsäureumsatz betrug 98,0 %.

Der auf ca. 25°C abgekühlte Reaktoraustrag wurde zur Säureabtrennung in einstufigen Mixer-Settler-Apparaturen mit einer 6 Gew%-igen Natronlauge (200 ml/h) und mit Wasser (100 ml/h) gewaschen.

Die weitgehend säurefreie organische Phase wurde in einer Destillationseinheit, bestehend aus einem Umlaufverdampfer, einer Kolonne mit 40 Glockenböden, einem Kondensator und einem Phasentrenngefäß, in eine Leichtsiederfraktion und ein Sumpfprodukt, das hauptsächlich n-Butylacrylat und Hochsieder enthielt, aufgetrennt. 107 g der Leichtsiederfraktion, hauptsächlich aus Butylacrylat (63,6 Gew%), Butylacetat (1,3 Gew%), Butanol (26 Gew%) und Dibutylether (0,5 Gew%) bestehend, wurde in die Veresterungskaskade rückgeführt. Das Rücklaufverhältnis betrug 10. Die Sumpftemperatur betrug 107 °C, die Kopftemperatur 80 °C bei 175 mbar. Die Stabilisierung der Destillationseinheit erfolgte durch Aufbringen von 50 g einer 1 Gew%-igen Lösung von Phenothiazin in n-Butylacrylat auf den Kondensator.

Aus dem Sumpfprodukt wurde in einer weiteren Destillationseinheit, bestehend aus einem Umlaufverdampfer, einer Dual-Flow Kolonne (30 Böden) und einem Kondensator, n-Butylacrylat in einer Reinheit von 99,9 % abgetrennt (Ausbeute 95 %). Der Zulauf erfolgte auf dem 10. Boden, die Sumpftemperatur betrug 110°C, die Kopftemperatur 80°C bei 105 mbar. Die Stabilisierung der Kolonne erfolgte mit Hydrochinonmonomethylether 0,1 Gew%-ig in n-Butylacrylat über den Rücklauf (15 ppm Hydrochinonmonomethylether, Rücklaufverhältnis 0,4) und durch Zugabe einer 1 Gew%-igen Hydrochinonmonomethylether-Lösung in n-Butylacrylat auf den 15. Boden.

Die bei der Säureabtrennung anfallenden wäßrigen Phasen wurden vereinigt, mit konzentrierter Schwefelsäure angesäuert (pH 1) und mit einem Gemische aus 120 g/h Destillat aus der Umesterung analog Beispiel 1 und 80 g n-Butylacrylat in einer dreistufigen Mixer-Settler-Apparatur extrahiert. Die acrylsäurehaltige organische Phase in der kein Dimethylaminoethanol nachgewiesen werden konnte (3 Gew% Acrylsäure) wurde in den ersten Veresterungsreaktor zurückgeführt.

Die Veresterung konnte mindestens 500 Stunden problemlos betrieben werden, ohne daß Dimethylaminoethylacrylat im Produkt nachweisbar war.

**Patentansprüche**

1. Verfahren zur Herstellung von höheren (Meth)acrylsäureestern durch Umesterung eines niederen (Meth)acrylsäureesters mit einem höheren Alkohol $R^2OH$, der entweder als $R^2$ einen $C_2$-$C_{12}$-Alkylrest mit mindestens einer $NR^3$ 2-Gruppe, in der $R^3$ ein $C_1$-$C_6$-Alkylrest ist und N auch Glied eines fünf- bis siebengliedrigen Ringes sein kann, trägt oder $R^3_2N(-CH_2CH_2-O)_y-H$, $R^3_2N(-CH(CH_3)-CH_2O)_y-H$ beziehungsweise $R^3_2N(-CH_2CH(CH_3)-O)_y-H$, worin y für eine ganze Zahl zwischen 1 und 4 steht, ist, in Gegenwart eines Stabilisators oder Stabilisatorgemisches und eines Katalysators oder Katalysatorgemischs, **dadurch gekennzeichnet, daß** man das freigesetzte niedere Alkanol $R^1OH$, wobei $R^1$ mindestens 1 Kohlenstoffatom weniger enthält als $R^2$, abtrennt und ohne weitere Reinigung zumindest teilweise der Herstellung des niederen (Meth)acrylsäureesters zuführt.

2. Verfahren zur Herstellung von höheren (Meth)acrylsäureestern durch Umesterung eines niederen (Meth)acrylsäureesters mit einem höheren Alkohol $R^2OH$, der ausgewählt ist aus 2-(Dimethylamino)-ethanol, 3-(Dimethylamino)-propanol, 4-(Dimethylamino)-butanol, 5-(Dimethylamino)-pentanol, 6-(Dimethylamino)-hexanol, 8-(Dimethylamino)-octanol, 10-(Dimethylamino)-decanol, 12-(Dimethylamino)-dodecanol, 2-(Diethylamino)-ethanol, 3-(Diethylamino)-propanol, 4-(Diethylamino)-butanol, 5-(Diethylamino)-pentanol, 6-(Diethylamino)-hexanol, 8-(Diethylamino)-octanol, 10-(Diethylamino)-decanol, 12-(Diethylamino)-dodecanol, 2-(Di-(*iso*-propyl)-amino)-ethanol, 3-(Di-(*iso*-propyl)-amino)-propanol, 4-(Di-(*iso*-propyl)-amino)-butanol, 5-(Di-(*iso*-propyl)-amino)-pentanol, 6-(Di-(*iso*-propyl)-amino)-hexanol, 8-(Di-(*iso*-propyl)-amino)-octanol, 10-(Di-(*iso*-propyl)-amino)-decanol, 12-(Di-(*iso*-propyl)-amino)-dodecanol, 2-(Dibutylamino)-ethanol, 3-(Dibutylamino)-propanol, 4-(Dibutylamino)-butanol, 5-(Dibutylamino)-pentanol, 6-(Dibutylamino)-hexanol, 8-(Dibutylamino)-octanol, 10-(Dibutylamino)-decanol, 12-(Dibutylamino)-dodecanol, 2-(Dihexylamino)-ethanol, 3-(Dihexylamino)-propanol, 4-(Dihexylamino)-butanol, 5-(Dihexylamino)-pentanol, 6-(Dihexylamino)-hexanol, 8-(Dihexylamino)-octanol, 10-(Dihexylamino)-decanol, 12-(Dihexylamino)-dodecanol, 2-(Methyl-ethyl-amino)-ethanol, 2-(Methylpropyl-amino)-ethanol, 2-(Methyl-*iso*-propyl-amino)-ethanol, 2-(Methyl-butyl-amino)-ethanol, 2-(Methyl-hexylamino)-ethanol, 2-(Methyl-octyl-amino)-ethanol, 2-(Ethyl-propyl-amino)-ethanol, 2-(Ethyl-*iso*-propyl-amino)-ethanol, 2-(Ethyl-butyl-amino)-ethanol, 2-(Ethyl-hexyl-amino)-ethanol, 2-(Ethyl-octyl-amino)-ethanol, 3-(Methyl-ethylamino)-propanol, 3-(Methyl-propyl-amino)-propanol, 3-(Methyl*iso*-propyl-amino)-propanol, 3-(Methyl-butyl-amino)-propanol, 3-(Methyl-hexyl-amino)-propanol, 3-(Methyl-octylamino)-propanol, 3-(Ethyl-propyl-amino)-propanol, 3-(Ethyl*iso*-propyl-amino)-propanol, 3-(Ethyl-butyl-amino)-propanol, 3-(Ethyl-hexyl-amino)-propanol, 3-(Ethyl-octylamino)-propanol, 4-(Methyl-ethyl-amino)-butanol, 4-(Methylpropyl-amino)-butanol, 4-(Methyl-*iso*-propyl-amino)-butanol, 4-(Methyl-butyl-amino)-butanol, 4-(Methyl-hexylamino)-butanol, 4-(Methyl-octyl-amino)-butanol, 4-(Ethyl-propyl-amino)-butanol, 4-(Ethyl-iso-propyl-amino)-butanol, 4-(Ethyl-butyl-amino)-butanol, 4-(Ethyl-hexyl-amino)-butanol, 4-(Ethyl-octyl-amino)-butanol, 2-(N-Piperidinyl)-ethanol, 3-(N-Piperidinyl)-propanol, 4-(N-Piperidinyl)-butanol, 5-(N-Piperidinyl)-pentanol, 6-(N-Piperidinyl)-hexanol, 8-(N-Piperidinyl)-octanol, 10-(N-Piperidinyl)-decanol, 12-(N-Piperidinyl)-dodecanol, 2-(N-Pyrrolidinyl)-ethanol, 3-(N-Pyrrolidinyl)-propanol, 4-(N-Pyrrolidinyl)-butanol, 5-(N-Pyrrolidinyl)-pentanol, 6-(N-Pyrrolidinyl)-hexanol, 8-(N-Pyrrolidinyl)-octanol, 10-(N-Pyrrolidinyl)-decanol, 12-(N-Pyrrolidinyl)-dodecanol, 2-(N-Morpholino)-ethanol, 3-(N-Morpholino)-propanol, 4-(N-Morpholino)-butanol, 5-(N-Morpholino)-pentanol, 6-(N-Morpholino)-hexanol, 8-(N-Morpholino)-octanol, 10-(N-Morpholino)-decanol, 12-(N-Morpholino)-dodecanol, 2-(N'-Methyl-N-Piperazinyl)-ethanol, 3-(N'-Methyl-N-Piperazinyl)-propanol, 4-(N'-Methyl-N-Piperazinyl)-butanol, 5-(N'-Methyl-N-Piperazinyl)-pentanol, 6-(N'-Methyl-N-Piperazinyl)-hexanol, 8-(N'-Methyl-N-Piperazinyl)-octanol, 10-(N'-Methyl-N-Piperazinyl)-decanol, 12-(N'-Methyl-N-Piperazinyl)-dodecanol, 2-(N'-Ethyl-N-Piperazinyl)-ethanol, 3-(N'-Ethyl-N-Piperazinyl)-propanol, 4-(N'-Ethyl-N-Piperazinyl)-butanol, 5-(N'-Ethyl-N-Piperazinyl)-pentanol, 6-(N'-Ethyl-N-Piperazinyl)-hexanol, 8-(N'-Ethyl-N-Piperazinyl)-octanol, 10-(N'-Ethyl-N-Piperazinyl)-decanol, 12-(N'-Ethyl-N-Piperazinyl)-dodecanol, 2-(N'-*iso*-Propyl-N-Piperazinyl)-ethanol, 3-(N'-*iso*-Propyl-N-Piperazinyl)-propanol, 4-(N'-*iso*-Propyl-N-Piperazinyl)-butanol, 5-(N'-*iso*-Propyl-N-Piperazinyl)-pentanol, 6-(N'-*iso*-Propyl-N-Piperazinyl)-hexanol, 8-(N'-*iso*-Propyl-N-Piperazinyl)-octanol, 10-(N'-*iso*-Propyl-N-Piperazinyl)-decanol, 12-(N'-*iso*-Propyl-N-Piperazinyl)-dodecanol oder ethoxylierte und/oder propoxylierte Alkohole sowie gemischt-ethoxylierte/propoxylierte Aminoalkohole, $R^3_2N(-CH_2CH_2-O)_y-H$ oder $R^3_2N(-CH(CH_3)-CH_2-O)_y-H$ beziehungsweise $R^3_2N(-CH_2-CH(CH_3)-O-)_y-H$, worin y für eine ganze Zahl zwischen 1 und 4 steht, in Gegenwart eines Stabilisators oder Stabilisatorgemisches und eines Katalysators oder Katalysatorgemischs, **dadurch gekennzeichnet, daß** man das freigesetzte niedere Alkanol $R^1OH$, das ausgewählt ist aus Methanol, Ethanol, n-Propanol, iso-Propanol, n-Butanol, iso-Butanol, 2-Butanol und tert.-Butanol, abtrennt und ohne weitere Reinigung zumindest teilweise der Herstellung des niederen (Meth)acrylsäureesters zuführt.

3. Verfahren nach Anspruch 2, **dadurch gekennzeichnet, daß** der höhere Alkohol ausgewählt ist aus Dimethylaminoethanol, Diethylaminoethanol, Di-n-butylaminoethanol, 3-Dimethylaminopropanol, 3-Diethylaminopropanol und 3-Di butylaminopropanol.

4. Verfahren nach einem der vorstehenden Ansprüche, **dadurch gekennzeichnet, daß** man das freigesetzte niedere Alkanol $R^1OH$ abtrennt und zumindest teilweise dem Aufarbeitungsprozeß der Herstellung des niederen (Meth) acrylsäureesters zuführt.

5. Verfahren nach einem der vorstehenden Ansprüche, **dadurch gekennzeichnet, daß** die Abtrennung des niederen Alkanols $R^1OH$ destillativ erfolgt.

6. Verfahren nach einem der vorstehenden Ansprüche, **dadurch gekennzeichnet, daß** das bei der Umesterung freigesetzte niedere Alkanol $R^1OH$ im wesentlichen zusammen mit niederem (Meth)acrylsäureester sowie gegebenenfalls höherem Alkohol $R^2OH$ abgetrennt wird.

7. Verfahren nach einem der vorstehenden Ansprüche, dadurch g-kennzeichnet, daß man das bei der Umesterung freigesetzte und abgetrennte, niederes Alkanol $R^1OH$ enthaltende Gemisch im Aufarbeitungsprozeß der Herstellung des niederen (Meth)acrylsäureesters mit einem (Meth)acrylsäure enthaltenden Strom in Kontakt bringt.

8. Verfahren nach Anspruch 7, **dadurch gekennzeichnet, daß** man das bei der Umesterung freigesetzte und abgetrennte, niederes Alkanol $R^1OH$ enthaltende Gemisch im Aufarbeitungsprozeß der Herstellung des niederen (Meth) acrylsäureesters zur Extraktion von (Meth)acrylsäure verwendet.

9. Verfahren nach einem der Ansprüche 7 oder 8, **dadurch gekennzeichnet, daß** man das bei der Umesterung freigesetzte und abgetrennte, niederes Alkanol $R^1OH$ enthaltende Gemisch im Aufarbeitungsprozeß der Herstellung des niederen (Meth)acrylsäureesters zur Extraktion von (Meth)acrylsäure aus angesäuertem Waschwasser verwendet.

10. Verfahren nach einem der Ansprüche 8 bis 9, **dadurch gekennzeichnet, daß** die Extraktion im Gegenstrom durchgeführt wird.

11. Verfahren nach einem der vorstehenden Ansprüche, **dadurch gekennzeichnet, daß** es kontinuierlich durchgeführt wird.

12. Verfahren nach einem der vorstehenden Ansprüche, **dadurch gekennzeichnet, daß** es sich bei dem niederen Alkanol $R^1OH$ um n-Butanol und bei dem niederen (Meth)acrylsäureester um (Meth)acrylsäure-n-butylester handelt.

13. Verfahren nach einem der vorstehenden Ansprüche, **dadurch gekennzeichnet, daß** es sich bei dem höheren Alkanol $R^2OH$ um 2-(N,N-Dimethylamino)-ethanol und bei dem höheren (Meth)acrylsäureester um 2-(N,N-Dimethylamino)-ethyl(meth)acrylat handelt.

## Claims

1. A process for the preparation of higher (meth)acrylates by transesterification of a lower (meth)acrylate with a higher alcohol $R^2OH$ which either carries, as $R^2$, a $C_2$-$C_{12}$-alkyl radical having at least one $NR^3_2$ group in which $R^3$ is $C_1$-$C_6$-alkyl and N may also be a member of a five- to seven-membered ring or is $R^3_2N(-CH_2CH_2-O)_y-H$, $R^3_2N(-CH(CH_3)-CH_2-O)_y-H$ or $R^3_2N(-CH_2CH(CH_3)-O)_y-H$, where y is an integer from 1 to 4, in the presence of a stabilizer or stabilizer mixture and of a catalyst or catalyst mixture, wherein the liberated lower alkanol $R^1OH$, where $R^1$ comprises at least 1 carbon atom less than $R^2$, is separated off and is fed at least partly to the preparation of the lower (meth) acrylate without further purification.

2. A process for the preparation of higher (meth)acrylates by transesterification of a lower (meth)acrylate with a higher alcohol $R^2OH$ which is selected from 2-(dimethylamino)ethanol, 3-(dimethylamino)propanol, 4-(dimethylamino)butanol, 5 -(dimethylamino)pentanol, 6-(dimethylamino)hexanol, 8-(dimethylamino)octanol, 10-(dimethylamino)decanol, 12-(dimethylamino)dodecanol, 2-(diethylamino)ethanol, 3-(diethylamino)propanol, 4-(diethylamino)butanol, 5-(diethylamino)pentanol, 6-(diethylamino)hexanol, 8-(diethylamino)octanol, 10-(diethylamino)decanol, 12-(diethylamino)dodecanol, 2-(di(*iso*propyl)amino)ethanol, 3-(di(*iso*propyl)amino)propanol, 4-(di(*iso*propyl)amino)-butanol, 5-(di(*iso*propyl)amino)pentanol, 6-(di(*iso*propyl)amino)hexanol, 8-(di(*iso*propyl)amino)octanol, 10-(di(*iso*propyl) amino)decanol, 12-(di(*iso*propyl)amino)-dodecanol, 2-(dibutylamino)ethanol, 3-(dibutylamino)propanol, 4-(dibutylamino)butanol, 5-(dibutylamino)pentanol, 6-(dibutylamino)hexanol, 8-(dibutylamino)octanol, 10-(dibutylamino) decanol, 12-(dibutylamino)dodecanol, 2-(dihexylamino)ethanol, 3-(dihexylamino)propanol, 4-(dihexylamino)butanol, 5-(dihexylamino)pentanol, 6-(dihexylamino)hexanol, 8-(dihexylamino)octanol, 10-(dihexylamino)decanol, 12-(dihexylamino)dodecanol, 2-(methylethylamino)ethanol, 2-(methylpropylamino)ethanol, 2-(methyl*iso*propylamino)-ethanol, 2-(methylbutylamino)ethanol, 2-(methylhexylamino)ethanol, 2-(methyloctylamino)ethanol, 2-(ethylpropylamino)ethanol, 2-(ethyl*iso*propylamino)ethanol, 2-(ethylbutylamino)ethanol, 2-(ethylhexylamino)ethanol, 2-(ethyloctylamino)ethanol, 3-(methylethylamino)propanol, 3-(methylpropylamino)propanol, 3-(methyl*iso*propylamino)-propanol, 3-(methylbutylamino)propanol, 3-(methylhexylamino)-propanol, 3-(methyloctylamino)propanol, 3-(ethylpropylamino)-propanol, 3-(ethyl*iso*propylamino)propanol, 3-(ethylbutylamino)propanol, 3-(ethylhexylamino)propanol, 3-(ethyloctylamino)propanol, 4-(methylethylamino)butanol, 4-(methylpropylamino)butanol, 4-(methyl*iso*propylamino)-butanol, 4-(methylbutylamino) butanol, 4-(methylhexylamino)butanol, 4-(methyloctylamino)butanol, 4-(ethylpropylamino)butanol, 4-(ethyl*iso*propylamino)butanol, 4-(ethylbutylamino)butanol, 4-(ethylhexylamino)butanol, 4-(ethyloctylamino)butanol, 2-(N-pipexidinyl)ethanol, 3-(N-piperidinyl)propanol, 4-(N-piperidinyl) butanol, 5-(N-piperidinyl)pentanol, 6-(N-piperidinyl)hexanol, 8-(N-piperidinyl)octanol, 10-(N-piperidinyl)decanol, 12-(N-piperidinyl)dodecanol, 2-(N-pyrrolidinyl)ethanol, 3-(N-pyrrolidinyl)propanol, 4-(N-pyrrolidinyl)butanol, 5-(N-pyrrolidinyl)pentanol, 6-(N-pyrrolidinyl)hexanol, 8-(N-pyrrolidinyl)octanol, 10-(N-pyrrolidinyl)decanol, 12-(N-pyrrolidinyl)dodecanol, 2-(N-morpholino)ethanol, 3-(N-morpholino)propanol, 4-(N-morpholino)butanol, 5-(N-morpholino) pentanol, 6-(N-morpholino)hexanol, 8-(N-morpholino)octanol, 10-(N-morpholino)decanol, 12-(N-morpholino)dodecanol, 2-(N'-methyl-N-piperazinyl)-ethanol, 3-(N'-methyl-N-piperazinyl)propanol, 4-(N'-methyl-N-piperazinyl) butanol, 5- (N' -methyl-N-pipexazinyl)pentanol, 6-(N'-methyl-N-piperazinyl)hexanol, 8-(N'-methyl-N-piperazinyl)octanol, 10-(N'-methyl-N-piperazinyl)decanol, 12-(N'-methyl-N-piperazinyl)-dodecanol, 2-(N'-ethyl-N-piperazinyl)ethanol, 3-(N'-ethyl-N-piperazinyl) propanol, 4-(N'-ethyl-N-piperazinyl)butanol, 5-(N'-ethyl-N-piperazinyl)pentanol, 6-(N'-ethyl-N-piperazinyl)hexanol, 8-(N'-ethyl-N-piperazinyl)octanol, 10-(N'-ethyl-N-piperazinyl)decanol, 12-(N'-ethyl-N-piperazinyl)dodecanol, 2-(N'-*iso*propyl-N-piperazinyl)ethanol, 3-(N'-*iso*propyl-N-piperazinyl)-propanol, 4-(N'-*iso*propyl-N-piperazinyl) butanol, 5-(N'-*iso*propyl-N-piperazinyl)pentanol, 6-(N'-*iso*propyl-N-piperazinyl)hexanol, 8-(N'-*iso*propyl-N-piperazinyl)octanol, 10-(N'-*iso*propyl-N-piperazinyl)decanol, 12-(N'-*iso*propyl-N-piperazinyl)dodecanol or ethoxylated and/or propoxylated alcohols and mixed ethoxylated/propoxylated amino alcohols, $R^3_2N(-CH_2CH_2-O)_y-H$ or $R^3_2N(-CH(CH_3)-CH_2-O)_y-H$ or $R^3_2N(-CH_2-CH(CH_3)-O-)_y-H$, where y is an integer from 1 to 4, in the presence of a stabilizer or stabilizer mixture and of a catalyst or catalyst mixture, wherein the liberated lower alkanol $R^1OH$, which is selected from methanol, ethanol, n-propanol, isopropanol, n-butanol, isobutanol, 2-butanol and tert-butanol, is separated off and is fed at least partly to the preparation of the lower (meth)acrylate without further purification.

**3.** The process according to claim 2, wherein the higher alcohol is selected from dimethylaminoethanol, diethylaminoethanol, di-n-butylaminoethanol, 3-dimethylaminopropanol, 3-diethylaminopropanol and 3-dibutylaminopropanol.

**4.** The process according to any of the preceding claims, wherein the liberated lower alkanol $R^1OH$ is separated off and is fed at least partly to the working-up process in the preparation of the lower (meth)acrylate.

**5.** The process according to any of the preceding claims, wherein the lower alkanol $R^1OH$ is separated off by distillation.

**6.** The process according to any of the preceding claims, wherein the lower alkanol $R^1OH$ liberated during the transesterification is separated off substantially together with lower (meth)acrylate and any higher alcohol $R^2OH$.

**7.** The process according to any of the preceding claims, wherein the mixture liberated during the transesterification, separated off and comprising lower alkanol $R^1OH$ is brought into contact with a (meth)acrylic acid-comprising stream in the working-up process in the preparation of the lower (meth)acrylate.

**8.** The process according to claim 7, wherein the mixture liberated during the transesterification, separated off and comprising lower alkanol $R^1OH$ is used in the working-up process in the preparation of the lower (meth)acrylate for the extraction of (meth)acrylic acid.

**9.** The process according to either of claims 7 and 8, wherein the mixture liberated during the transesterification, separated off and comprising lower alkanol $R^1OH$ is used in the working-up process in the preparation of the lower (meth) acrylate for the extraction of (meth)acrylic acid from acidified washwater.

**10.** The process according to either of claims 8 and 9, wherein the extraction is carried out countercurrently.

**11.** The process according to any of the preceding claims, which is carried out continuously.

**12.** The process according to any of the preceding claims, wherein the lower alkanol $R^1OH$ is n-butanol and the lower (meth)acrylate is n-butyl (meth)acrylate.

**13.** The process according to any of the preceding claims, wherein the higher alkanol $R^2OH$ is 2-(N,N-dimethylamino) ethanol and the higher (meth)acrylate is 2-(N,N-dimethylamino)ethyl (meth) acrylate.

## Revendications

**1.** Procédé pour la préparation d'esters supérieurs de l'acide (méth)acrylique par transestérification d'un ester inférieur de l'acide (méth)acrylique avec un alcool supérieur $R^2OH$, qui porte soit, comme $R^2$, un radical $C_2$-$C_{12}$-alkyle comprenant au moins un groupe $NR^3_2$, dans lequel $R^3$ représente un radical $C_1$-$C_6$-alkyle et N peut également être un chaînon d'un cycle de cinq à sept chaînons, soit $R^3_2N(-CH_2CH_2-O)_y-H$, $R^3_2N(-CH(CH_3)-CH_2-O)_y-H$ ou, selon le cas, $R^3_2N(-CH_2CH(CH_3)-O)_y-H$, y valant un nombre entier entre 1 et 4, en présence d'un stabilisateur ou d'un mélange de stabilisateurs et d'un catalyseur ou d'un mélange de catalyseurs, **caractérisé en ce que** qu'on sépare l'alcanol inférieur $R^1OH$ libéré, $R^1$ contenant au moins un atome de carbone de moins que $R^2$, et on l'alimente sans autre purification au moins partiellement dans la préparation de l'ester inférieur de l'acide (méth)acrylique.

**2.** Procédé pour la préparation d'esters supérieurs de l'acide (méth)acrylique par transestérification d'un ester inférieur de l'acide (méth)acrylique avec un alcool supérieur $R^2OH$, qui est choisi parmi le 2-(diméthylamino)-éthanol, le 3-(diméthylamino)-propanol, le 4-(diméthylamino)-butanol, le 5-(diméthylamino)-pentanol, le 6-(diméthylamino)-hexanol, le 8-(diméthylamino)-octanol, le 10-(diméthylamino)-décanol, le 12-(diméthylamino)-dodécanol, le 2-(diéthylamino)-éthanol, le 3-(diéthylamino)-propanol, le 4-(diéthylamino)-butanol, le 5-(diéthylamino)-pentanol, le 6-(diéthylamino)-hexanol, le 8-(diéthylamino)-octanol, le 10-(diéthylamino)-décanol, le 12-(diéthylamino)-dodécanol, le 2-(di-(isopropyl)-amino)-éthanol, le 3-(di-(isopropyl)-amino)-propanol, le 4-(di-(isopropyl)-amino)-butanol, le 5-(di-(isopropyl)-amino)-pentanol, le 6-(di-(isopropyl)-amino)-hexanol, le 8-(di-(isopropyl)-amino)-octanol, le 10-(di-(isopropyl)-amino)-décanol, le 12-(di-(isopropyl)-amino)-dodécanol, le 2-(dibutylamino)-éthanol, le 3-(dibutylamino)-propanol, le 4-(dibutylamino)-butanol, le 5-(dibutylamino)-pentanol, le 6-(dibutylamino)-hexanol, le 8-(dibutylamino)-octanol, le 10-(dibutylamino)-décanol, le 12-(dibutylamino)-dodécanol, le 2-(dihexylamino)-éthanol, le 3-(dihexylamino)-propanol, le 4-(dihexylamino)-butanol, le 5-(dihexylamino)-pentanol, le 6-(dihexylamino)-hexanol, le 8-(dihexylamino)-octanol, le 10-(dihexylamino)-décanol, le 12-(dihexylamino)-dodécanol, le 2-(méthyléthylamino)-éthanol, le 2-(méthylpropylamino)-éthanol, le 2-(méthylisopropylamino)-éthanol, le 2-(méthylbutylamino)-éthanol, le 2-(méthylhexylamino)-éthanol, le 2-(méthyloctylamino)-éthanol, le 2-(éthylpropylamino)-éthanol, le 2-(éthylisopropylamino)-éthanol, le 2-(éthylbutylamino)-éthanol, le 2-(éthylhexylamino)-éthanol, le 2-(éthyloctylamino)-éthanol, le 3-(méthyléthylamino)-propanol, le 3-(méthylpropylamino)-propanol, le 3-(méthylisopropylamino)-propanol, le 3-(méthylbutylamino)-propanol, le 3-(méthylhexylamino)-propanol, le 3-(méthyloctylamino)-propanol, le 3-(éthylpropylamino)-propanol, le 3-(éthylisopropylamino)-propanol, le 3-(éthylbutylamino)-propanol, le 3-(éthylhexylamino)-propanol, le 3-(éthyloctylamino)-propanol, le 4-(méthyléthylamino)-butanol, le 4-(méthylpropylamino)-butanol, le 4-(méthylisopropylamino)-butanol, le 4-(méthylbutylamino)-butanol, le 4-(méthylhexylamino)-butanol, le 4-(méthyloctylamino)-butanol, le 4-(éthylpropylamino)-butanol, le 4-(éthylisoprapylamino)-butanol, le 4-(éthylbutylamino)-butanol, le 4-(éthylhexylamino)-butanol, le 4-(éthylactylamino)-butanol, le 2-(N-pipéridinyl)-éthanol, le 3-(N-pipéridinyl)-propanol, le 4-(N-pipéridinyl)-butanol, le 5-(N-pipéridinyl)-pentanol, le 6-(N-pipéridinyl)-hexanol, le 8-(N-pipéridinyl)-octanol, le 10-(N-pipéridinyl)-décanol, le 12-(N-pipéridinyl)-dodécanol, le 2-(N-pyrralidinyl)-éthanol, le 3-(N-pyrrolidinyl)-propanol, le 4-(N-pyrrolidinyl)-butanol, le 5-(N-pyrrolidinyl)-pentanol, le 5-(N-pyrrolidinyl)-hexanal, le 8-(N-pyrrolidinyl)-octanol, le 10-(N-pyrrolidinyl)-décanol, le 12-(N-pyrrolidinyl)-dodécanol, le 2-(N-morpholino)-éthanal, le 3-(N-morpholino)-propanol, le 4-(N-morpholino)-butanol, le 5-(N-morpholino)-pentanol, le 6-(N-morpholino)-hexanol, le 8-(N-morpholino)-octanol, le 10-(N-morpholino)-décanol, le 12-(N-morpholino)-dodécanol, le 2-(N'-méthyl-N-pipérazinyl)-éthanol, le 3-(N'-méthyl-N-pipérazinyl)-propanol, le 4-(N'-méthyl-N-pipérazinyl)-butanol, le 5-(N'-méthyl-N-pipérazinyl)-pentanol, le 6-(N'-méthyl-N-pipérazinyl)-hexanol, le 8-(N'-méthyl-N-pipérazinyl)-octanol, le 10-(N'-méthyl-N-pipérazinyl)-décanol, le 12-(N'-méthyl-N-pipérazinyl)-dodécanol, le 2-(N'-éthyl-N-pipérazinyl)-éthanol, le 3-(N'-éthyl-N-pipérazinyl)-propanol, le 4-(N'-éthyl-N-pipérazinyl)-butanol, le 5-(N'-éthyl-N-pipérazinyl)-pentanol, le 6-(N'-éthyl-N-pipérazinyl)-hexanol, le 8-(N'-éthyl-N-pipérazinyl)-octanol, le 10-(N'-éthyl-N-pipérazinyl)-décanol, le 12-(N'-éthyl-N-pipérazinyl)-dodécanol, le 2-(N'-iso-propyl-N-pipérazinyl)-éthanol, le 3-(N'-iso-propyl-N-pipérazinyl)-propanol, le 4-(N'-iso-propyl-N-pipérazinyl)-butanol, le 5-(N'-iso-propyl-N-pipérazinyl)-pentanol, le 6-(N'-iso-propyl-N-pipérazinyl)-hexanol, le 8-(N'-iso-propyl-N-pipérazinyl)-octanol, le 10-(N'-iso-propyl-N-pipérazinyl)-décanol, le 12-(N'-iso-propyl-N-pipérazinyl)-dodécanol ou les alcools éthoxylés et/ou propoxylés ainsi que les aminoalcools éthoxylés/propoxylés mixtes, le $R^3_2N(-CH_2CH_2-O)_y-H$ ou le $R^3_2N(-CH(CH_3)-CH_2-O)_y-H$ ou, selon le cas, le $R^3_2N(-CH_2-CH(CH_3)-O-)_y-H$, y valant un nombre entier entre 1 et 4, en présence

d'un stabilisateur ou d'un mélange de stabilisateurs et d'un catalyseur ou d'un mélange de catalyseurs, **caractérisé en ce que** qu'on sépare l'alcanol inférieur $R^1OH$ libéré, qui est choisi parmi le méthanol, l'éthanol, le n-propanol, l'iso-propanol, le n-butanol, l'iso-butanol, le 2-butanol et le tert-butanol, et on l'alimente sans autre purification au moins partiellement dans la préparation de l'ester inférieur de l'acide (méth)acrylique.

**3.** Procédé selon la revendication 2, **caractérisé en ce que** l'alcool supérieur est choisi parmi le diméthylaminoéthanol, le diéthylaminoéthanol, le di-n-butylaminoéthanol, le 3-diméthylaminopropanol, le 3-diéthylaminopropanol et le 3-dibutylaminopropanol.

**4.** Procédé selon l'une quelconque des revendications précédentes, **caractérisé en ce qu'**on sépare l'alcanol inférieur $R^1OH$ libéré et on l'alimente au moins partiellement dans le procédé de traitement de la préparation de l'ester inférieur de l'acide (méth)acrylique.

**5.** Procédé selon l'une quelconque des revendications précédentes, **caractérisé en ce que** la séparation de l'alcanol inférieur $R^1OH$ a lieu par distillation.

**6.** Procédé selon l'une quelconque des revendications précédentes, **caractérisé en ce que** l'alcanol inférieur $R^1OH$ libéré lors de la transestérification est séparé sensiblement en même temps que l'ester inférieur de l'acide (méth) acrylique ainsi que le cas échéant l'alcool supérieur $R^2OH$.

**7.** Procédé selon l'une quelconque des revendications précédentes, **caractérisé en ce que** qu'on met en contact le mélange contenant l'alcanol inférieur $R^1OH$ libéré lors de la transestérification et séparé dans le procédé de traitement de la préparation de l'ester inférieur de l'acide (méth)acrylique avec un flux contenant de l'acide (méth)acrylique.

**8.** Procédé selon la revendication 7, **caractérisé en ce que** qu'on utilise le mélange contenant l'alcanol inférieur $R^1OH$ libéré lors de la transestérification et séparé dans le procédé de traitement de la préparation de l'ester inférieur de l'acide (méth)acrylique pour l'extraction de l'acide (méth)acrylique.

**9.** Procédé selon l'une quelconque des revendications 7 ou 8, **caractérisé en ce que** qu'on utilise le mélange contenant l'alcanol inférieur $R^1OH$ libéré lors de la transestérification et séparé dans le procédé de traitement de la préparation de l'ester inférieur de l'acide (méth)acrylique pour l'extraction de l'acide (méth)acrylique de l'eau de lavage acidifiée.

**10.** Procédé selon l'une quelconque des revendications 8 à 9, **caractérisé en ce que** l'extraction est réalisée à contre-courant.

**11.** Procédé selon l'une quelconque des revendications précédentes, **caractérisé en ce qu'**il est réalisé en continu.

**12.** Procédé selon l'une quelconque des revendications précédentes, **caractérisé en ce qu'**il s'agit, pour l'alcanol inférieur $R^1OH$, de n-butanol et, pour l'ester inférieur de l'acide (méth)acrylique, d'ester n-butylique de l'acide (méth) acrylique.

**13.** Procédé selon l'une quelconque des revendications précédentes, **caractérisé en ce qu'**il s'agit, pour l'alcanol supérieur $R^2OH$, de 2-(N,N-diméthylamino)-éthanol et, pour l'ester supérieur de l'acide (méth)acrylique, de (méth) acrylate de 2-(N,N-diméthylamino)-éthyle.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2323328 C2
- DE 19510891 A
- DE 19851983 A
- DE 19536178 A
- WO 9923060 A
- US 4280010 A
- DE 19604252 A
- DE 19604253 A
- US 3868410 A
- WO 0027789 A
- DE 19547485 A
- DE 19547459 A
- DE 19701737 A
- CN 1058390
- CN 1063678
- EP 298867 B1
- EP 960877 A2
- DE OS2008618 A
- EP 906902 A
- EP 906902 A2
- US 2406561 A
- DE OS2145283 A
- EP 210907 B1
- DE 2317226 A
- EP 143639 A2
- EP 736510 A1
- EP 160427 A2
- DE 10127938
- DE 2323328
- DE 10127939
- DE 19851983 A1


### In der Beschreibung aufgeführte Nicht-Patentliteratur


- Kirk Othmer, Encyclopedia of Chemical Technology. 1994, 301-302
- Organikum. VEB Deutscher Verlag, 1988, 506